# EUROPEAN PATENT APPLICATION

(11) **EP 3 095 870 A1**
(43) Date of publication of application: **23.11.2016**
(21) Application number: 15202060.8
(22) Date of filing: 22.12.2015
(51) Int. Cl.: C12N 15/82

(54) **METHODS FOR THE IN PLANTA TRANSFORMATION OF PLANTS AND MANUFACTURING PROCESSES AND PRODUCTS BASED AND OBTAINABLE THEREFROM**

(30) Priority: 19.05.2015 DE 102015006335; 05.11.2015 DE 102015014252
(71) Applicant: KWS SAAT SE, 37574 Einbeck (DE)
(72) Inventor: Nießen, Markus, 30880 Laatzen (DE); Harling, Hinrich, 37120 Bovenden (DE); Martin-Ortigosa, Susana, 37574 Einbeck (DE)

(57) **Abstract**

The present invention relates to *in planta* transformation methods for plants or plant materials with a genetic construct, wherein at least one meristematic cell of a meristematic tissue of an immature inflorescence able to differentiate into a gamete of a pollen or of an ovule is exposed and then transformed, wherein the transformation can be performed to yield a stable integration or to yield a transient introduction of a genetic construct of interest. Further provided are methods for manufacturing a transgenic plant, or methods for manufacturing a genetically manipulated plant based on the *in planta* transformation methods according to the present invention. In addition, there is provided a plant or a progeny thereof manufactured according to the transformation and/or manufacturing methods according to the present invention.

## Description

### Technical Field

The present invention relates to *in planta* transformation methods for plants or plant materials with a genetic construct, wherein at least one meristematic cell of a meristematic tissue of an immature inflorescence able to differentiate into a gamete of a pollen or of an ovule is exposed and then transformed, wherein the transformation can be performed to yield a stable integration or to yield a transient introduction of a genetic construct of interest. Further provided are methods for manufacturing a transgenic plant, or methods for manufacturing a genetically manipulated plant based on the *in planta* transformation methods according to the present invention. In addition, there is provided a plant or a progeny thereof manufactured according to the transformation and/or manufacturing methods according to the present invention. Finally, there is provided the use of the inventive methods and plants for the manufacture of a transgenic or genetically manipulated plant or plant material.

### Background of the Invention

Over the last decades many techniques for the genetic transformation of plants have been developed. All said methods have the ultimate goal to obtain a transgenic plant containing in all or part of the cells a foreign nucleic acid comprising a gene or a feature of interest, in the case of a gene a transgene, but with the rise of new genome engineering techniques, for example the CRISPR/Cas technology (see e.g. US 8,697,359 B1, EP 2 800 811 A1 or WO 2013/142578 A1) also the insertion of ribonucleic acids or sequences encoding the same and of amino acid sequences becomes more and more relevant. In the case of nucleic acid molecules encoding a transgene optionally further elements, the stable integration into a plants' genome, particularly the nuclear genome, but also into the genome of e.g. plant plastids, is one of the major aims of the preceding transformation process to yield a stable "knock-in", but likewise a stably inheritable "knock-out" of a gene or region of interest. For other applications, for instance when designing experiments using genome editing tools like CRISPR/Cas, the transient expression of the construct of interest to be transformed or transfected might be of interest to just temporarily influence the genetic material of a plant, i.e. the genome, or the transcriptome, i.e. all RNA material, thereof in a controlled way.

Currently, there exists a variety of plant transformation methods to introduce genetic material in the form of a genetic construct into a plant cell of interest. One preferred technique is transformation with *Agrobacterium* spp. which has been used for decades for a variety of different plant materials. *Agrobacterium* transformation, however, is much better established for dicotyledonous plants than for monocotyledonous plants, the latter comprising economically important plants e.g. in the family of Poacea/Graminaceae including *inter alia* plants like maize/corn, wheat, barley, rye, sorghum or sugar cane. Over the recent years, some progress could be achieved for other plant transformation methods by choosing direct delivery techniques for introducing genetic material into a plant cell, e.g. by choosing direct delivery techniques ranging from polyethylene glycol (PEG) treatment of protoplasts (Potrykus *et al.* 1985), procedures like electroporation (D'Halluin *et al.,* 1992), microinjection (Neuhaus *et al.,* 1987),silicon carbide fiber whisker technology (Kaeppler *et al.,* 1992), viral vector mediated approaches (Gelvin, Nature Biotechnology 23, "Viral-mediated plant transformation gets a boost", 684-685 (2005)) and particle bombardment (see e.g. Sood et al., 2011, Biologia Plantarum, 55, 1-15). For all the above methods, it is still mandatory to have an explanted plant material, including embryogenic calli or suspension cells, scutellar tissue, calli of different types, including type 1 and type 2 and organogenic type 3 calli, protoplasts, immature embryos, or green tissue which is then treated *in vitro*,i.e. *ex planta,* and not within the living plant.

One exemplary transformation process for corn is *Agrobacterium* mediated transformation of immature embryos of defined genotypes through somatic embryogenesis. The process of regenerating a plant from the *in vitro* transformed material is, however, time consuming, expensive and the efficiency and the parameters to follow are strongly genotype dependent. In addition, only few genotypes are suitable for genetic transformation, requiring backcrosses of the generated transgenic material with elite varieties which is time consuming and costly.

US 6,603,061 B1 discloses method of transforming a corn plant cell or plant tissue using an *Agrobacterium* mediated process by inoculating a transformable plant cell or tissue from a corn plant with *Agrobacterium* containing at least one genetic component capable of being transferred to the plant cell or tissue in an inoculation media containing an effective amount of at least one antibiotic that inhibits or suppresses the growth of *Agrobacterium.* There are several publications in the scientific literature about *Agrobacterium* mediated transformation of flowers like the floral dip methodologies, routinely applied to *Arabidopsis,* but also to crops like wheat (Clough and Bent 1998; Zale et al. 2009).Said approaches, however, intrinsically require the cumbersome regeneration of the transformed plant cell or tissue to obtain a transgenic or genetically modified plant.

Pace and Dupuis ("Gene transfer to maize male reproductive structure by particle bombardment", 1993, Plant Cell reports, 12: 607-611.) discloses a method for transforming tassel primordia of maize using particle bombardment for transient expression of foreign genes. The method according to Pace and Dupuis relies on the bombardment *in vitro* and all experiments are performed with explanted cells and no reproductive plant or plant material was or can be obtained from this approach. Pareddy and Greyson (1985) published a method wherein maize tassels (1 month old) of the genotype OH43 were dissected and bombarded in tassel maturation media. The bombardment was done at 1350 psi with 1.6 µm gold particles and the tassels were bombarded 4 times. The tassels cultured *in vitro* matured, but produced a low amount of anthers comparing to the greenhouse plants. Transient expression in the tassels was assayed. Most of it was detected in the outer glumes, only some in the stamen primordia. After 1 month of *in vitro* culture anthers were produced and tested for gus activity. Only 0.5% of all anthers showed gus activity, mostly in the vascular tissue of the anther. No analyses were done on pollen and no pollinations were performed. Therefore, there is still need for improving this method to obtain sufficient amount of transformed cells having a high transformation rate.

In wheat, a bombardment technology called "micro-targeting" was used to transform isolated immature spikes of wheat (Leduc et al. 1994). Those spikes were cultured in osmoticum medium to increase the transient expression. Even though around 80% of the particles were targeted to the L1 layer of cells (the outer layer that will produce all the epidermis tissue of the ear) and approximately 20% targeted L2 cells (that will produce reproductive organs). The authors obtained "multicellular sectors showing transgene expression" 12 days after bombardment. Those sectors were found in primordia of vegetative and reproductive organs like anthers. They describe this methodology as a possible "direct transformation of sporogenic tissues". However, there is no follow-up of the technology, especially no follow up *in planta* or for other important crop plants.

The bombardment of plant cells with a nucleic acid of interest represents another process for transformation and is known since the late 1980's (Taylor and Fauquet, 2002) for manipulating the genome of plants (at that time) recalcitrant to transformation via *Agrobacterium,* including *inter alia* cereals (Klein et al 1987; Taylor and Fauquet, 2002). The transformation via particle bombardment uses a microprojectile of metal covered with the gene of interest, which is then shot onto the target cells using an equipment known as "gene gun" (Sandford et al. 1987) at high velocity fast enough (∼1500 km/h) to penetrate the cell wall of a target tissue, but not harsh enough to cause cell death. The precipitated nucleic acid or the nucleic acid construct on the at least one microprojectile is released into the cell after bombardment, and integrated into the genome. The acceleration of microprojectiles is accomplished by a high voltage electrical discharge or compressed gas (helium). This technique was progressively further developed, e.g. for inserting more than one gene of interest into target cells (Chen et al. 1998; Wu et al. 2002). Still, several physical parameters correlated with the biolistic equipment such as pressure, distance of the macro and micro-carrier flight, and vacuum, must be optimized for a successful transformation. Another crucial parameter is the target cell or tissue itself, as this target site has to be readily accessible, as well as the nucleic acid construct of interest to be introduced.

Despite transformation methods based on biological approaches, like *Agrobacterium* transformation or viral vector mediated plant transformation, and methods based on physical delivery methods, like particle bombardment or microinjection, have evolved as prominent techniques for introducing genetic material into a plant cell or tissue of interest over the last years, there are still several problems associated therewith hampering the broad approach for both transient as well as stable introduction of a construct of interest in any kind of plant to be transformed.

Helenius et al. ("Gene delivery into intact plants using the Helios™ Gene Gun", Plant Molecular Biology Reporter, 2000, 18 (3):287-288) discloses a particle bombardment as physical method for transient transformation of gene constructs into intact plant tissue of model plants like *Arabidposis* and tobacco. There are, however, no examples concerning either the achievement of stable transformation of the genetic construct or any advice of how to use the technology for major crop plants in plant organs other than leaves. Furthermore, the document is silent as to a method for directly obtaining a genetically modified seed directly in a modified plant obviating the need for *in vitro* cultivation or further crossing steps, as the target tissue is not a meristematic tissue.

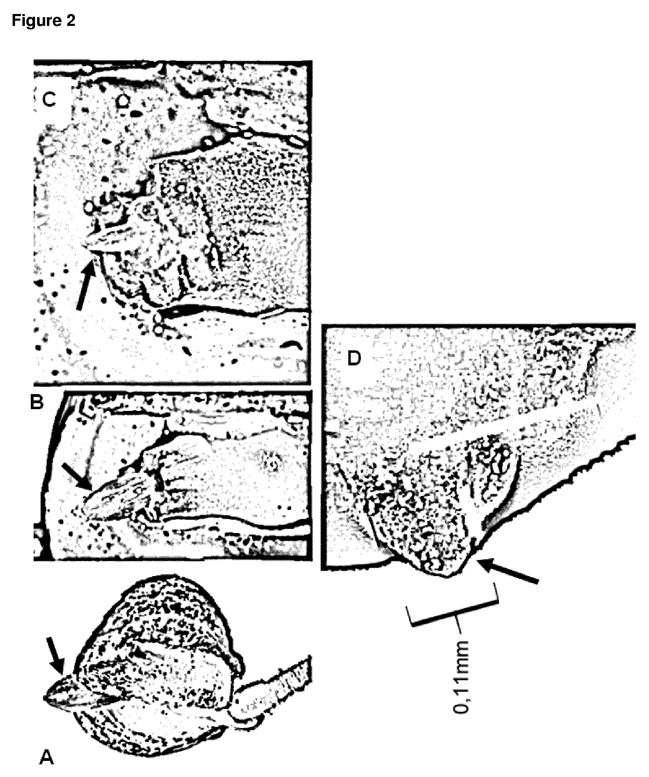

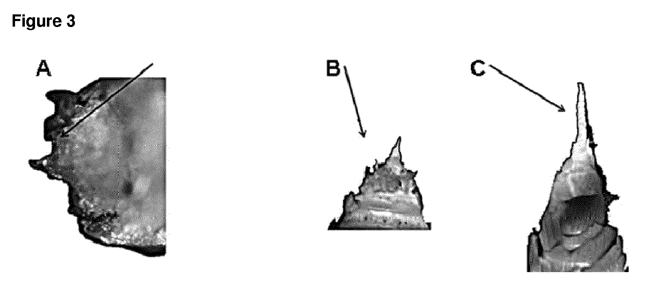

Thus, it would represent a preferable targeting strategy to specifically transform the meristematic tissue of a plant of interest, as a targeted genetic manipulation effected for at least one meristematic cell provides the advantage of inheriting said manipulation to the progeny of said not yet fully differentiated cells. To this end, however, a meristematic target structure has to be exposed in a suitable way to avoid the destruction of the plant organism and/or the target structure of interest. As elucidated in **Fig. 2** and **3****,** the meristem in seedlings and older maize plants is completely surrounded by leaves so that the meristematic tissue has to be prepared before exposing it to a further treatment. The principle of providing a meristematic plant tissue as target structure is disclosed in DE 10 2015 006 335.9 and DE 10 2015 014 252.6.

A transformation method that would be genotype and *in vitro* culture independent, i.e. which could be performed directly *in planta* in a plant of interest, would thus be desirable to save time and resources. The possibility of adapting the transformation process to a variety of monocot and dicot plants of economic interest would be highly beneficial. Furthermore, there is a need in providing plant transformation methods, which are useful for both the transient and the stable introduction of a construct of interest in a plant. Finally, it remains an outstanding need to create stable or transient transformation of a plant cell or a plant material *in planta* which directly yields transformed meristematic structures in a plant within an inflorescence meristematic cell or tissue, as this would pave the way for the direct acquisition of plant reproductive material directly in the modified plant and not only from the progeny thereof or after cumbersome and expensive *in vitro* cultivation.

### Summary of the Invention

The primary object of the present invention was thus the provision of methods for the genotype independent *in planta* transformation of a plant or a plant material, which is suitable for both, the transient as well as the stable introduction/integration of a genetic construct of interest into at least one meristematic cell of a meristematic tissue belonging to the immature inflorescence of a plant. Furthermore, it was an object to provide methods for the manufacturing of a transgenic or a genetically manipulated plant based on an *in planta* approach for transforming a plant or plant material. Finally it was an object to obtain a plant or a progeny of a plant manufactured by the methods according to the present invention or plant cells or a plant material, or derivatives thereof or to provide and use a plant transformed by the methods according to the present invention for the manufacture of a transgenic or genetically manipulated plant or plant material.

The primary object has been achieved by providing in one aspect a method for the *in planta* transformation of a plant or plant material with a genetic construct of interest comprising the following steps: (i) providing a plant comprising at least one meristematic cell of a meristematic tissue of an immature inflorescence, wherein the meristematic cell is able to differentiate into a gamete of a pollen or of an ovule; (ii) exposing the at least one meristematic cell of the meristematic tissue of the immature inflorescence; (iii) providing a genetic construct of interest; and (iv) transforming the at least one meristematic cell of the meristematic tissue of the immature inflorescence by introducing the genetic construct of interest under suitable conditions to allow the functional integration of the genetic construct of interest into the at least one meristematic cell of the meristematic tissue of the immature inflorescence.

In one embodiment, the transformation is performed *in vitro* culture free.

In another embodiment, the functional integration of the genetic construct of interest into the at least one meristematic cell of the meristematic tissue of the immature inflorescence of step (iv) of the above aspect is performed as (a) a stable integration so that the integrated genetic construct of interest, or a part thereof, is heritable to a progeny, preferably whereby a transformed gamete of the pollen or of the ovule is generated from the at least one transformed meristematic cell and the transformed gamete of the pollen or of the ovule is fused to a zygote from which the progeny comprising the genetic construct of interest is developed; as transgene, or (b) a transient introduction allowing a targeted genetic manipulation of the at least one meristematic cell of the meristematic tissue of the immature inflorescence through the genetic construct of interest or products thereof, wherein the targeted genetic manipulation but not the genetic construct of interest, or a part thereof, is heritable to a progeny, preferably whereby a genetic manipulated gamete of the pollen or of the ovule is generated from the transformed at least one meristematic cell and the genetic manipulated gamete of the pollen or of the ovule is fused to a zygote from which the progeny comprising the targeted genetic manipulation is developed.

In another embodiment of the above aspect, the method comprises an additional step (v) comprising determining the functional integration of the genetic construct of interest.

In yet another embodiment of the above aspect according to the present invention, the introduction of the genetic construct of interest according to step (iv) of the above aspect is conducted using a means selected from the group consisting of a device suitable for particle bombardment, including a gene gun, transformation, including transformation using *Agrobacterium spp.* or using a viral vector, microinjection, electroporation, whisker technology, including silicon carbide whisker technology, and transfection, or a combination thereof.

There is provided a further embodiment, wherein step (ii) of the above aspect comprises the production of an artificially introduced window in the region, where the meristematic tissue of the immature inflorescence is located, preferably in the shoot axis, in particular in the axil, halm, culm or stem of the plant.

In yet another embodiment this aspect according to the present invention the plant or the plant material is or is part of a monocotyledonous (monocot) plant, or is or is part of a dicotyledonous (dicot) plant.

In a further embodiment according to the above aspect according to the present invention the plant or the plant material is or is part of a plant from a species selected from the group consisting of *Hordeum vulgare, Hordeum bulbusom, Sorghum bicolor, Saccharum officinarium, Zea mays, Setaria italica, Oryza minuta, Oriza sativa, Oryza australiensis, Oryza alta, Triticum aestivum, Secale cereale, Malus domestica, Brachypodium distachyon, Hordeum marinum, Aegilops tauschii, Daucus glochidiatus, Beta vulgaris, Daucus pusillus, Daucus muricatus, Daucus carota, Eucalyptus grandis, Nicotiana sylvestris, Nicotiana tomentosiformis, Nicotiana tabacum, Solanum lycopersicum, Solanum tuberosum, Coffea canephora, Vitis vinifera, Erythrante guttata, Genlisea aurea, Cucumis sativus, Morus notabilis, Arabidopsis arenosa, Arabidopsis lyrata, Arabidopsis thaliana, Crucihimalaya himalaica, Crucihimalaya wallichii, Cardamine flexuosa, Lepidium virginicum, Capsella bursa pastoris, Olmarabidopsis pumila, Arabis hirsute, Brassica napus, Brassica oeleracia, Brassica rapa, Raphanus sativus, Brassica juncea, Brassica nigra, Eruca vesicaria subsp. sativa, Citrus sinensis, Jatropha curcas,Populus trichocarpa, Medicago truncatula, Cicer yamashitae, Cicer bijugum, Cicer arietinum, Cicer reticulatum, Cicer judaicum, Cajanus cajanifolius, Cajanus scarabaeoides, Phaseolus vulgaris, Glycine max, Gossypium sp., Astragalus sinicus, Lotus japonicas, Torenia fournieri, Allium cepa, Allium fistulosum, Allium sativum,* and *Allium tuberosum.*

In another embodiment according to the first aspect of the present invention, the immature inflorescence is a male inflorescence (tassel) of a *Zea mays* plant.

In yet another embodiment of the present invention, the introduction of the genetic construct of interest as defined in step (iv) according to the above aspect is performed at a developmental stage, either during the stamen initiation process or before spikelets are formed on the male inflorescence.

In a second aspect according to the present invention there is provided a method for manufacturing a transgenic plant comprising the following steps: (i) providing an *in planta* transformed plant or plant material transformed by a method according to the first aspect of the present invention; (ii) cultivating the plant or plant material under suitable conditions until the developmental stage of maturity of the inflorescence is achieved, (iii) allowing the transformed gamete of the pollen or of the ovule generated from the transformed at least one meristematic cell to fuse to a zygote comprising the genetic construct of interest as transgene; and (iv) developing the transgenic plant from the zygote.

In a third aspect according to the present invention there is provided a method for manufacturing a genetically manipulated plant comprising the following steps: (i) providing an *in planta* transformed plant or plant material transformed by a method according the first aspect of the present invention detailed above; (ii) cultivating the plant or plant material under suitable conditions until the developmental stage of maturity of the inflorescence is achieved; (iii) allowing the genetically manipulated gamete of the pollen or of the ovule generated from the transformed at least one meristematic cell to fuse to a zygote comprising the targeted genetic manipulation; and (iv) developing the genetically manipulated plant from the zygote.

In still another aspect according to the present invention there is provided a plant manufactured by the manufacturing methods of the second or third aspect of the present invention, or plant cells, a plant material, or derivatives or a progeny thereof.

In yet another aspect according to the present invention there is provided a plant, preferably a *Zea mays* plant, comprising an artificially introduced window in a region, where a meristematic tissue of an immature inflorescence is located, preferably in the axil at the culm of the plant, preferably wherein the plant is a plant *in planta* transformed by a method according to the first aspect according to the present invention.

There is provided a further aspect according to the present invention which is directed to the use of a plant comprising an artificially introduced window in a region, where a meristematic tissue of an immature inflorescence is located, preferably in the axil at the culm of the plant, preferably wherein the plant is a plant *in planta* transformed by a method according to the first aspect according to the present invention, or of a plant *in planta* transformed by a method according to the first aspect according to the present invention, for the manufacture of a transgenic or genetically manipulated plant, plant cell or plant material.

Further aspects and embodiments of the present invention can be derived from the subsequent detailed description, the drawings as well as the attached set of claims.

### Brief Description of the Drawings

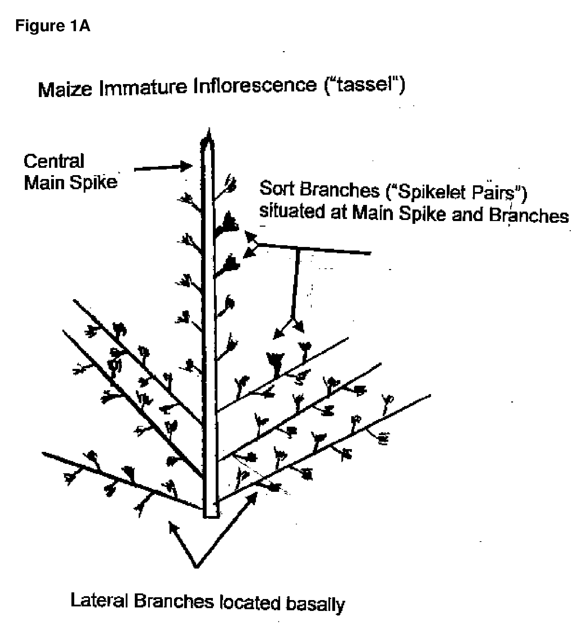

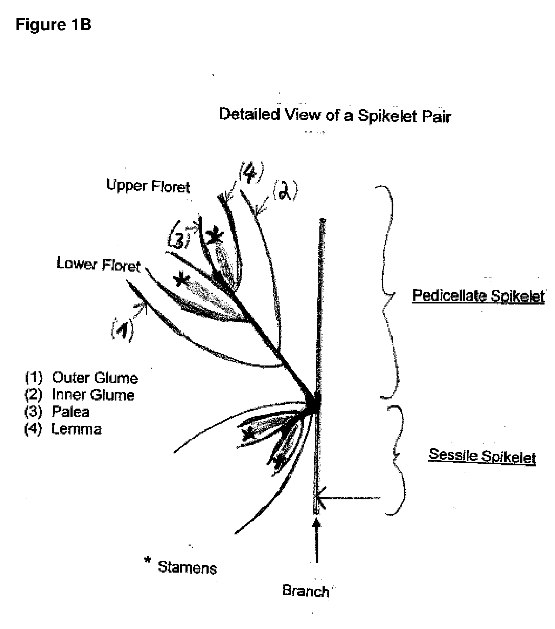

Figure 1 (Fig. 1) shows a schematic diagram of the maize immature inflorescence, the tassel and illustrates details of a spikelet pair. Fig. 1 (A) shows the male inflorescence (tassel) consisting of a central main spike. At the base, long lateral branches branching off the main branch can be found. Short branches called "spikelet pairs" cover both the main spike as well as the lateral branches. Fig. 1 (B) shows a detailed view of a spikelet pair from a normal maize tassel. The spikelet pair is situated at a branch (lateral or main branch). The Sessile Spikelet of the spikelet pair originates from the base where it is attached to whilst the Pedicellate Spikelet is situated on a pedicel. Each spikelet contains two florets, the upper floret and the lower floret enclosed by two glumes, the inner glume and the outer glume. In more detail, each of said florets consists of lemma, palea and two lodicules and three stamens (indicated with a * within each floret).

Figure 2 A-D (Fig. 2 A-D) shows dissected meristems in maize seedlings. As the meristem is completely covered by leaves in seedlings, it has to be dissected first to be exposed for the further treatment. To this end, the surrounding tissue structures have to be removed to expose the meristem (indicated with an arrow in Fig. 2).

Figure 3 A-C (Fig. 3 A-C) shows dissected meristems in older maize plants. As the meristem in older plants is completely surrounded by leaves, as for the seedlings shown in Fig. 2, it has to be dissected first without extracting or isolating it to be exposed for a further treatment. To this end the proximal leaves have to be removed to expose the meristem (indicated with an arrow in the respective Figures A to C). Age of the plants in Figure 3: (A) 8 days, (B) 11 days, (C) 46 days.

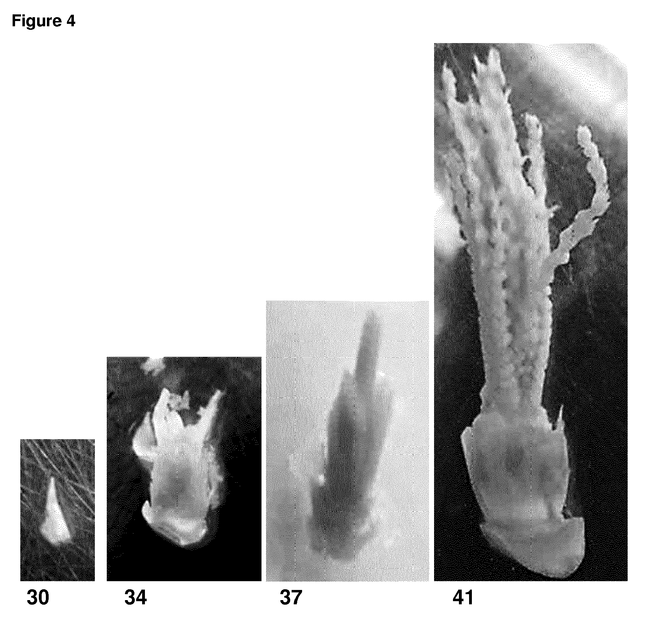

Figure 4 (Fig. 4) shows the tassel development in maize at different days calculated after sowing, i.e. at days 30, 34, 37 and 41, respectively.

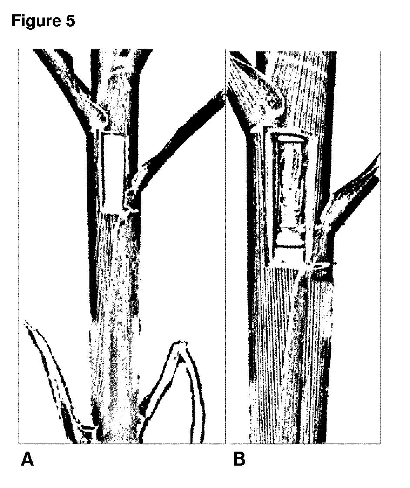

Figure 5 (Fig. 5) exemplary shows the window opening and the tassel exposure for a maize A188 plant. (A) Removal one by one of leaf tissue. (B) Exposed tassel after window opening.

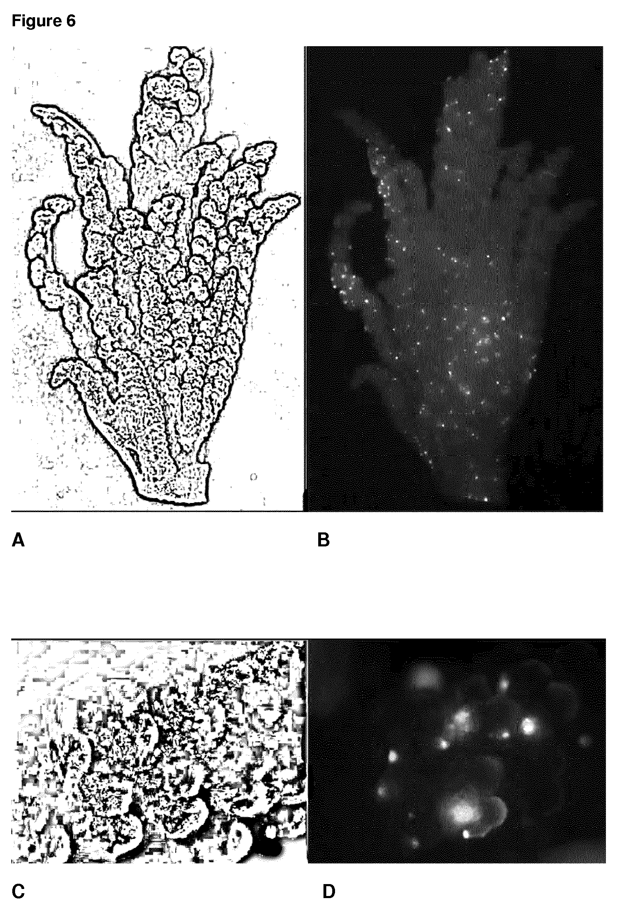

Figure 6 A-D (Fig. 6 A-D) shows immature tassels detached from the plant and bombarded with a PDS-1000/He gene gun with a genetic construct driving the expression of a red fluorescent protein (RFP). Expression in immature tassels was observed 1 day after bombardment. (A) and (B) whole tassel, (B) fluorescence channel detection; (A) bright field picture of the same structure. White spots and regions in (B) correspond to red fluorescence indicative of expression of the RFP encoded by the genetic construct and transformed into the tassel. (C) and (D) Close up view of the immature spikelets. (D) Again, white spots and regions in (D) correspond to red spots detected corresponding to the expression of the RFP in the immature spikelet.

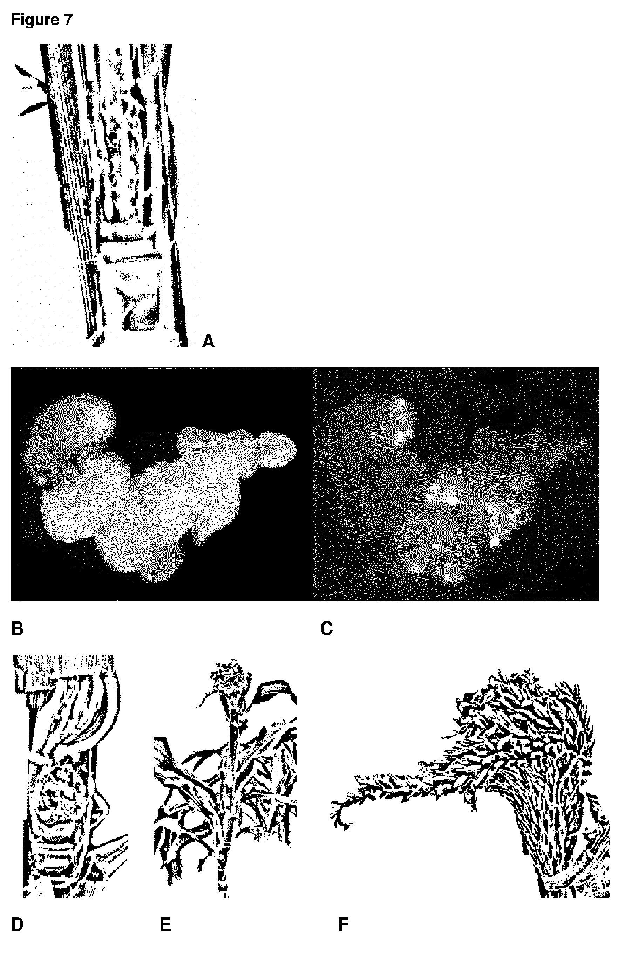

Figure 7 A-F (Fig. 7 A-F) shows the results of *in planta* bombardment with a construct encoding a red fluorescent protein (RFP). (A) Artificial window opened in the axil of a maize plant exposing the immature tassel. (B) and (C) A piece of tassel 1 day after bombardment showing transient expression of the RFP in immature spikelets (see Fig. 7 (C) - white spots corresponding to RFP expression as visualized via fluorescence imaging; (B) bright field picture of the same structure. (D): development of the plant after window cutting and bombardment. (E) and (F): further development *in planta* yielding a fertile tassel.

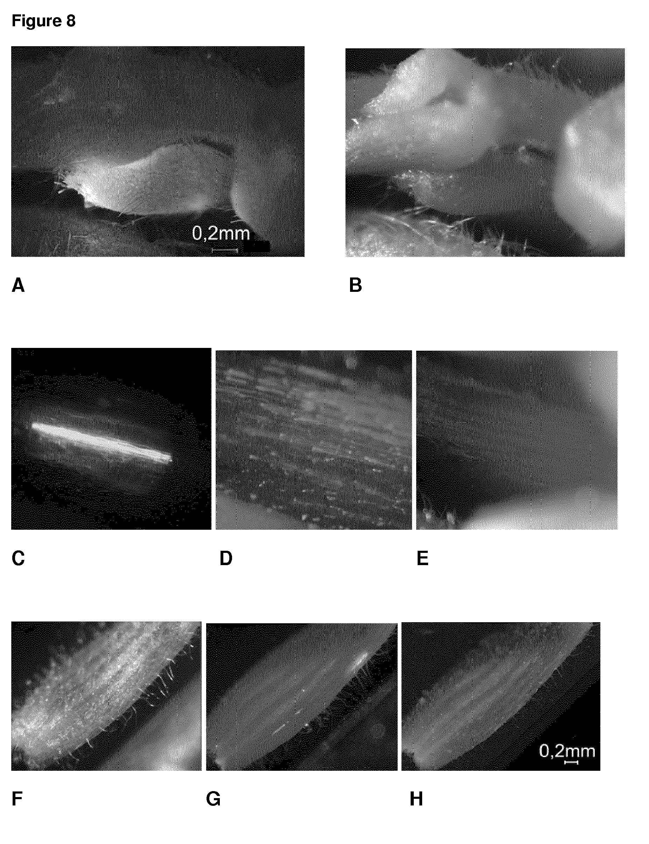

Figure 8 A-H (Fig. 8 A-H) shows an example for stable transformation (red fluorescent protein (RFP) encoding construct) as achieved by the methods of the present invention. (A) and (B): Picture of a spikelet 7 days after bombardment. (A) Red fluorescence channel visualizing RFP expression, here shown as bright white areas. (B) Bright field picture of the same structure as in (A). (C) to (E) Visualization of a cell of a glume of a spikelet 16 days after bombardment. (C) Red fluorescence channel, (D) green fluorescence channel, (D) bright field. The white structure in (C) corresponds to the red fluorescence observed. (F) to (H) shows the a glume transformed with a genetic construct encoding a RFP 28 days after bombardment. (F) Bright field, (G) red fluorescence channel with the bright white area corresponding to the detected red fluorescence; (H) green fluorescence channel.

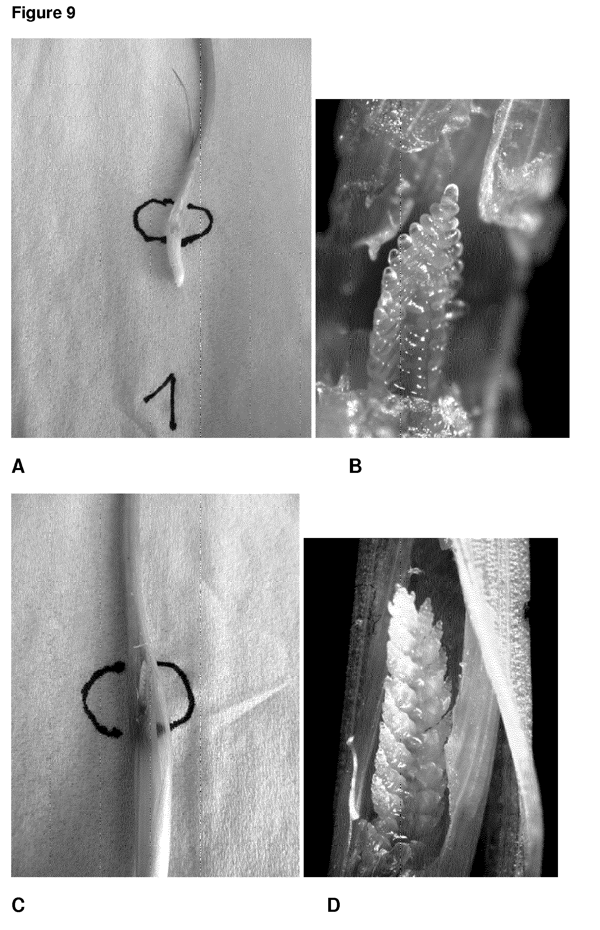

Figure 9 (Fig. 9 A-D) shows the localization of a wheat immature inflorescence in two different wheat cultivars. Fig, 9 (A) shows a shoot of a wheat plant 1. The circle on the paper in which the plant is located indicated the location of the meristem at the bottom of the stem, Fig. 9 (B) is an enlarged view of the meristem of plant 1 in exposed form, Fig. 9 (C) shows a shoot of a wheat plant 2. The circle on the paper on which the plant is located indicated the location of the meristem. In this cultivar, the meristem is located higher and big enough so that it can easily be detected with the eye. Fig. 9 (D) is an enlarged view of the meristem of plant 2 in exposed form.

### Definitions

As used herein the term *"in planta"* or *"in planta* transformation" means that the actual transformation process, i.e. the introduction of a genetic construct according to the present disclosure, is achieved by transforming a plant, plant cell or plant material which is still connected with the living plant it is derived from. Optionally, said *in planta* transformation can be conducted with a plant, plant cell, plant tissue or plant material that was specifically prepared or exposed for the transformation process still being in connection with the living plant. Said term is thus used to discriminate the respective methods from *in vitro ex planta* transformation methods, wherein a plant, plant cell, plant tissue or plant material is first dissected from its natural environment for subsequently being transformed *in vitro* or *ex vivo*/*ex planta.*

The term *"in vitro* culture free" as used according to the present disclosure thus implies that no *in vitro* culture is necessary for the respective process step. This implies that the respective process does directly take place *in planta* and not in an explanted or dissected plant cell, tissue, organ or material. The present invention in certain embodiments can comprise *in vitro* steps, e.g. analytical steps for analyzing the stable integration or the transient introduction of a genetic construct of interest according to the present disclosure. *In vitro* steps can also occur to cultivate a specific transformed plant material, if desired, depending on the desired plant, plant cell, tissue, organ or material to be obtained. Whenever the term *"in vitro* culture free" is used, the respective transformation method or the manufacturing process or a product obtainable therefrom, however, can completely proceed *in planta* without necessitating an *in vitro* culture step for propagating a plant cell, tissue or material.

The term "construct", especially "genetic construct" or "recombinant construct" (used interchangeably herein) as used herein refers to a construct comprising, *inter alia,* plasmids or plasmid vectors, cosmids, artificial yeast- or bacterial artificial chromosomes (YACs and BACs), phagemides, bacterial phage based vectors, an expression cassette, isolated single-stranded or double-stranded nucleic acid sequences, comprising DNA and RNA sequences, or amino acid sequences, viral vectors, including modified viruses, and a combination or a mixture thereof, for introduction or transformation, transfection or transduction into a target cell or plant, plant cell, tissue, organ or material according to the present disclosure. A recombinant construct according to the present invention can comprise an effector domain, either in the form of a nucleic acid or an amino acid sequence, wherein an effector domain represents a molecule, which can exert an effect in a target cell and includes a transgene, an single-stranded or double-stranded RNA molecule, including a guideRNA, a miRNA or an siRNA, or an amino acid sequences, including, *inter alia,* an enzyme or a catalytically active fragment thereof, a binding protein, an antibody, a transcription factor, a nuclease, preferably a site specific nuclease, and the like. Furthermore, the recombinant construct can comprise regulatory sequences and/or localization sequences. The recombinant construct can be integrated into a vector, including a plasmid vector, and/or it can be present isolated from a vector structure, for example, in the form of a polypeptide sequence or as a non-vector connected single-stranded or double-stranded nucleic acid. After its introduction, e.g. by transformation, the genetic construct can either persist extrachromosomally, i.e. non integrated into the genome of the target cell, for example in the form of a double-stranded or single-stranded DNA, a double-stranded or single-stranded RNA or as an amino acid sequence. Alternatively, the genetic construct, or parts thereof, according to the present disclosure can be stably integrated into the genome of a target cell, including the nuclear genome or further genetic elements of a target cell, including the genome of plastids like mitochondria or chloroplasts. The term "plasmid vector" as used in this connection refers to a genetic construct originally obtained from a plasmid. A plasmid usually refers to a circular autonomously replicating extrachromosomal element in the form of a double-stranded nucleic acid sequence. In the field of genetic engineering these plasmids are routinely subjected to targeted modifications by inserting, for example, genes encoding a resistance against an antibiotic or an herbicide, a gene encoding a target nucleic acid sequence, a localization sequence, a regulatory sequence, a tag sequence, a marker gene, including an antibiotic marker or a fluorescent marker, and the like. The structural components of the original plasmid, like the origin of replication, are maintained. According to certain embodiments of the present invention, the localization sequence can comprise a nuclear localization sequence, a plastid localization sequence, preferably a mitochondrion localization sequence or a chloroplast localization sequence. Said localization sequences are available to the skilled person in the field of plant biotechnology. A variety of plasmid vectors for use in different target cells of interest is commercially available and the modification thereof is known to the skilled person in the respective field.

The term "genetically modified" or "genetic manipulation" or "genetic(ally) manipulated" is used in a broad sense herein and means any modification of a nucleic acid sequence or an amino acid sequence, a target cell, tissue, organ or organism, which is accomplished by human intervention, either directly or indirectly, to influence the endogenous genetic material or the transciptome or the proteinome of a target cell, tissue, organ or organism to modify it in a purposive way so that it differs from its state as found without human intervention. The human intervention can either take place *in vitro* or *in vivo*/*in planta,* or also both. Further modifications can be included, for example, one or more point mutation(s), e.g. for targeted protein engineering or for codon optimization, deletion(s), and one or more insertion(s) or deletion(s) of at least one nucleic acid or amino acid molecule (including also homologous recombination), modification of a nucleic acid or an amino acid sequence, or a combination thereof. The terms shall also comprise a nucleic acid molecule or an amino acid molecule or a host cell or an organism, including a plant or a plant material thereof which is/are similar to a comparable sequence, organism or material as occurring in nature, but which have been constructed by at least one step of purposive manipulation.

A "targeted genetic manipulation" as used herein is thus the result of a "genetic manipulation", which is effected in a targeted way, i.e. at a specific position in a target cell and under the specific suitable circumstances to achieve a desired effect in at least one cell, preferably a plant cell, to be manipulated.

The term "transgenic" as used according to the present disclosure refers to a plant, plant cell, tissue, organ or material which comprises a gene or a genetic construct, comprising a "transgene" that has been transferred into the plant, the plant cell, tissue organ or material by natural means or by means of genetic engineering techniques from another organism. The term "transgene" comprises a nucleic acid sequence, including DNA or RNA, or an amino acid sequence, or a combination or mixture thereof. Therefore, the term "transgene" is not restricted to a sequence commonly identified as "gene", i.e. a sequence encoding protein. It can also refer, for example, to a non-protein encoding DNA or RNA sequence. Therefore, the term "transgenic" generally implies that the respective nucleic acid or amino acid sequence is not naturally present in the respective target cell, including a plant, plant cell, tissue, organ or material. The terms "transgene" or "transgenic" as used herein thus refer to a nucleic acid sequence or an amino acid sequence that is taken from the genome of one organism, or produced synthetically, and which is then introduced into another organism, in a transient or a stable way, by artificial techniques of molecular biology, genetics and the like.

The term "plant" or "plant cell" as used herein refers to a plant organism, a plant organ, differentiated and undifferentiated plant tissues, plant cells, seeds, and derivatives and progeny thereof. Plant cells include without limitation, for example, cells from seeds, from mature and immature embryos, meristematic tissues, seedlings, callus tissues in different differentiation states, leaves, flowers, roots, shoots, gametophytes, sporophytes, pollen and microspores, protoplasts, macroalgae and microalgae. The different plant cells can either be haploid, diploid or multiploid.

A "plant material" as used herein refers to any material which can be obtained from a plant during any developmental stage. The plant material can be obtained either *in planta* or from an *in vitro* culture of the plant or a plant tissue or organ thereof. The term thus comprises plant cells, tissues and organs as well as developed plant structures as well as sub-cellular components like nucleic acids, polypeptides and all chemical plant substances or metabolites which can be found within a plant cell or compartment and/or which can be produced by the plant, or which can be obtained from an extract of any plant cell, tissue or a plant in any developmental stage. The term also comprises a derivative of the plant material, e.g. a protoplast, derived from at least one plant cell comprised by the plant material. The term therefore also comprises meristematic cells or a meristematic tissue of a plant.

The term "transient introduction" as used herein refers to the transient introduction of at least one recombinant construct according to the present disclosure into a plant target structure, for example, a plant cell, wherein the at least one recombinant construct is introduced under suitable reaction conditions so that no integration of the at least one recombinant construct into the endogenous nucleic acid material of a target plant structure, i.e. the plant genome as a whole, occurs, so that the at least one recombinant construct will not be integrated into the endogenous DNA of the target cell. As a consequence, in the case of transient introduction, the introduced genetic construct will not be inherited to a progeny of the plant target structure, for example the plant cell. The at least one recombinant or genetic construct or the products resulting from transcription or translation thereof are only present temporarily, i.e. in a transient way, in constitutive or inducible form, and thus can only be active in the target cell for exerting their effect for a limited time. Therefore, the at least one genetic or recombinant construct introduced via transient introduction will not be heritable to the progeny of a cell. The effect which a recombinant construct introduced in a transient way will cause, can, however, potentially be inherited to the progeny of the target cell.

The term "stable integration" or "stably integrated" as used herein, refers to the stable integration of at least one recombinant or genetic construct according to the present disclosure. The integration can either take place into the nuclear genome or any other genomic extra-nuclear material within a plant compartment of interest, e.g. a mitochondrium. A stably integrated at least one recombinant construct will thus be heritable to the progeny of a thus modified target cell. Depending on the nature of the genetic construct, all or part of the genetic construct will be stably integrated, as the genetic construct may comprise several regions of interest comprising a target region to be stably integrated as well as further regions, *inter* alia, needed for the transport, delivery, maintenance, and the correct localization of the genetic construct within a plant cell, which regions, however, will not themselves be integrated, but serve as cargo for the region of interest to be stably integrated as it is known to the skilled person. The stable integration of at least one genetic construct according to the present disclosure into at least one meristematic cell or tissue will consequently lead to the inheritance of the thus modified genomic region of the plant target structure to the progeny of the modified cell through all developmental stages of said at least one meristematic cell, which can be favorable for approaches, where a targeted genetic modification in and the yield of the final cell type resulting from the differentiation and development of the at least one meristematic cell is desired. Achieving a stable integration into at least one meristematic cell of the immature inflorescence of a plant can thus lead to the stable inheritance of the introduced genetic feature into the gamete of the pollen or of the ovule developmentally resulting from the at least one meristematic cell of the immature inflorescence.

The term "particle bombardment" as used herein, also named "biolistic transfection" or "microparticle-mediated gene transfer", refers to a physical delivery method for transferring a coated microparticle or nanoparticle comprising a nucleic acid or a genetic construct of interest into a target cell or tissue. The micro or nanoparticle functions as projectile and is fired on the target structure of interest under high pressure using a suitable device, often called gene-gun. The transformation via particle bombardment uses a microprojectile of metal covered with the gene of interest, which is then shot onto the target cells using an equipment known as "gene gun" (Sandford et al. 1987) at high velocity fast enough (∼1500 km/h) to penetrate the cell wall of a target tissue, but not harsh enough to cause cell death. For protoplasts, which have their cell wall entirely removed, the conditions are different logically. The precipitated nucleic acid or the genetic construct on the at least one microprojectile is released into the cell after bombardment, and integrated into the genome. The acceleration of microprojectiles is accomplished by a high voltage electrical discharge or compressed gas (helium). Concerning the metal particles used it is mandatory that they are non-toxic, non-reactive, and that they have a lower diameter than the target cell. The most commonly used are gold or tungsten. There is plenty of information publicly available from the manufacturers and providers of gene-guns and associated system concerning their general use.

The term "derivative" or "descendant" or "progeny" as used herein in the context of a plant or plant cell or plant material according to the present application relates to the descendants of such a plant or plant cell or plant material which result from natural reproductive propagation including sexual and asexual propagation. It is well known to the person having skill in the art that said propagation can lead to the introduction of mutations into the genome of an organism resulting from natural phenomena which results in a descendant or progeny, which is genomically different to the parental plant or plant cell or plant material, however, still belongs to the same genus/species and possesses the same characteristics as the parental recombinant host cell. Such derivatives or descendants or progeny resulting from natural phenomena during reproduction or regeneration are thus comprised by the term present disclosure.

The term "vector" as used herein refers to a means of transport to deliver a genetic or a recombinant construct according to the present disclosure into a target cell, tissue, organ or plant. A vector thus comprises nucleic acid sequences, optionally comprising sequences like regulatory sequences or localization sequences for delivery, either directly or indirectly, into a target cell of interest or into a plant target structure in the desired cellular compartment of a plant. A vector can also be used to introduce an amino acid sequence into a target cell or target structure. Usually, a vector as used herein can be a plasmid vector. The term "direct introduction" implies that the desired target cell or target structure containing a nucleic acid sequence to be modified according to the present disclosure is directly transformed or transduced or transfected into the specific target cell of interest, where the material delivered with the vector will exert it effect. The term "indirect introduction" implies that the introduction is achieved into a structure, for example, cells of leaves or cells of plant organs or tissues, which do not themselves represent the actual target cell or structure of interest to be transformed, but those structures serve as basis for the systemic spread and transfer of the vector, preferably comprising a genetic construct according to the present disclosure to the actual plant target structure, for example, a meristematic cell or tissue. In case the term "vector" is used in the context of transfecting amino acid sequences into a target cell the term "vector" implies suitable agents for peptide or protein transfection, like for example ionic lipid mixtures. In the context of the introduction of nucleic acid material, the term "vector" cannot only imply plasmid vectors but also suitable carrier materials which can serve as basis for the introduction of nucleic acid or amino acid sequence delivery into a target cell of interest, for example by means of particle bombardment. Said carrier material comprises, *inter alia,* gold or tungsten particles. Finally, the term "vector" also implies the use of viral vectors for the introduction of at least one genetic construct according to the present disclosure like, for example, modified viruses for example derived from the following virus strains: Maize Streak Virus (MSV), Barley Stripe Mosaic Virus (BSMV), Brome Mosaic virus (BMV), Maize stripe virus (MSpV), Maize rayado fino virus (MYDV), Maize yellow dwarf virus (MYDV), Maize dwarf mosaic virus (MDMV),and bacterial vectors, like for example *Agrobacterium* spp., like for example *Agrobacterium tumefaciens.* Finally, the term "vector" also implies suitable transport agents for introducing linear nucleic acid sequences (single or double-stranded) into a target cell. The usual production processing and use of vectors as used according to the present disclosure can be accomplished by the person skilled in the art having knowledge of the present disclosure.

The term "target region", "target site", "target structure", "target construct", "target nucleic acid" or "target cell/tissue/organism" as used herein refers to a target which can be any genomic region within a target cell, but it also refers to extrachromosomal DNA or RNA, including mRNA, of a target cell or organism, or any structural element on or within a target cell, where a modification or manipulation is desired. The term "target region", "target site", "target structure", "target construct", "target nuclec acid" or "target cell/tissue/organism" is thus not restricted to genomic regions encoding a gene, i.e. a region encoding the information for being transcribed to an mRNA.

"Complementary" or "complementarity" as used herein describes the relationship of two DNA or RNA nucleic acid regions. Defined by the nucleobases of the DNA or RNA two nucleic acid regions can hybridize to each other in accordance with the lock-and-key model. To this end the principles of Watson-Crick base pairing have the basis adenine and thymine/uracil as well as guanine and cytosine, respectively, as complementary basis apply. Furthermore, also non-Watson-Crick pairing, like reverse-Watson-Crick, Hoogsteen, reverse-Hoogsteen and Wobble pairing are comprised by the term "complementary" as used herein as long as the respective base pairs can build hydrogen bonding to each other, i.e. two different nucleic acid strands can hybridize to each other based on said complementarity.

The term "regulatory sequence" as used herein refers to a nucleic acid or amino acid sequence, which can direct the transcription and/or translation and/or modification of a nucleic acid sequence of interest.

The term "nucleic acid (molecule)" or "nucleic acid construct "or "nucleic acid sequence" as used herein refers to natural and synthetic deoxyribonucleic acids (DNA) or ribonucleic acid (RNA) sequences, which can optionally comprise synthetic nucleic acid analoga. A nucleic acid according to the present disclosure can optionally be codon optimized. Codon optimization implies that the codon usage of a DNA or RNA is adapted to that of a cell or organism of interest to improve the transcription rate of said recombinant nucleic acid in the cell or organism of interest. The skilled person is well aware of the fact that a target nucleic acid can be modified at one position due to the codon degeneracy, whereas this modification will still lead to the same amino acid sequence at that position after translation, which is achieved by codon optimization to take into consideration the species-specific codon usage of a target cell or organism. Nucleic acid sequences according to the present application can carry specific codon optimization for the following non limiting list of organisms: *Hordeum vulgare, Sorghum bicolor, Secale cereale,* Triticale, *Saccharum officinarium, Zea mays, Setaria italic, Oryza sativa, Oryza minuta, Oryza australiensis, Oryza alta, Triticum aestivum, Triticum durum, Hordeum bulbosum, Brachypodium distachyon, Hordeum marinum, Aegilops tauschii, Malus domestica, Beta vulgaris, Helianthus annuus, Daucus glochidiatus, Daucus pusillus, Daucus muricatus, Daucus carota, Eucalyptus grandis, Erythranthe guttata, Genlisea aurea, Nicotiana sylvestris, Nicotiana tabacum, Nicotiana tomentosiformis, Solanum lycopersicum, Solanum tuberosum, Coffea canephora, Vitis vinifera, Cucumis sativus, Morus notabilis, Arabidopsis thaliana, Arabidopsis lyrata, Arabidopsis arenosa, Crucihimalaya himalaica, Crucihimalaya wallichii, Cardamine flexuosa, Lepidium virginicum, Capsella bursa-pastoris, Olmarabidopsis pumila, Arabis hirsuta, Brassica napus, Brassica oleracea, Brassica rapa, Brassica juncacea, Brassica nigra, Raphanus sativus, Eruca vesicaria sativa, Citrus sinensis, Jatropha curcas, Glycine max, Gossypium ssp.* or *Populus trichocarpa.*

The terms "protein", "polypeptide" and "enzyme" are used interchangeably herein. The term "amino acid" or "amino acid sequence" or "amino acid molecule" comprises any natural or chemically synthesized protein, peptide, polypeptide and enzyme or a modified protein, peptide, polypeptide and enzyme, wherein the term "modified" comprises any chemical or enzymatic modification of the protein, peptide, polypeptide and enzyme.

### Detailed Description

According to a first aspect of the present invention, there is provided a method for the *in planta* transformation of a plant or plant material with a genetic construct of interest comprising the following steps: (i) providing a plant comprising at least one meristematic cell of a meristematic tissue of an immature inflorescence, wherein the meristematic cell is able to differentiate into a gamete of a pollen or of an ovule; (ii) exposing the at least one meristematic cell of the meristematic tissue of the immature inflorescence; (iii) providing a genetic construct of interest; and (iv) transforming the at least one meristematic cell of the meristematic tissue of the immature inflorescence by introducing the genetic construct of interest under suitable conditions to allow the functional integration of the genetic construct of interest into the at least one meristematic cell of the meristematic tissue of the immature inflorescence.

The methods and uses as provided by the present invention and the plant products obtainable therefrom offers an advanced alternative to the current protocols of transforming *inter alia* the immature inflorescences, e.g. of corn, *in planta,* producing, for example, transgenic pollen that can be used for pollination, obtaining directly transgenic T1 seeds. Therefore, the methods as provided by the state of the art necessarily have to proceed through *in vitro* culture, irrespective of whether a stable or a transient modification of the plant is intended. The process according to the present invention is thus fast, genotype independent and *in vitro* culture free, if desired. Consequently, a transformed plant or plant material can be directly obtained from the plant being modified without cumbersome crossings and cultivation and screening of the F1 progeny. The methods as disclosed herein can be adapted to other flowers or inflorescences of other crops based on the disclosure provided herein.

As illustrated in **Figure 1****,** the immature inflorescence to be targeted can be the male inflorescence of corn, the tassel. The basic structure of the tassel inflorescence is the spikelet. Two types of spikelets, sessile and pedicellate spikelets, develop in the different branches of the tassel. Each spikelet covers with an outer and inner glume two individual flowers that consist of other two covers, the lemma and the palea that are protecting three stamens, the filament and the anther that will produce pollen, i.e. around 2-25 million pollen grains per tassel. The transformation methods according to the present invention should be done preferably during the stamen initiation process, introducing the foreign DNA in the L2 layer of cells of the stamen which is advantageous as those cells will end up producing transgenic pollen.

"Meristematic cells" as referred to according to the present disclosure belong to a tissue type within a plant which is also referred to as meristem or cambium or formative tissue. Like stem cells in animal organisms, meristematic cells of plants representing undifferentiated cells have the intrinsic capability to develop and differentiate into specialized cell types, depending on genetic predisposition and further environmental and developmental factors. In plant organisms, meristems are not only present during the embryo development, but they can be found during the whole life cycle of a plant so that a targeted genetic modification of meristematic cells or tissues according to the present disclosure is not restricted to plant embryos or seedlings, but it can rather also be conducted in larger seedlings and more matured plants, for example when targeting meristems which build the basis for the reproductive plant organs, for example the tassel or corn cob in maize.

According to one embodiment according to the various aspects according to the present disclosure the at least one meristematic cell is a mature or immature plant cell of a plant embryo or seedling or of a plant comprising at least one meristematic cell or meristematic tissue.

According to one specific embodiment according to the various aspects of the present disclosure, at least one a meristematic cell of a meristematic tissue of an immature inflorescence, wherein the meristematic cell is able to differentiate into a gamete of a pollen or of an ovule. As it is known to the skilled person in the field of plant sciences or plant biotechnology, the inflorescence of a seed plant is part of the shoot axis and involved in flower formation. Furthermore, it is known that there are unisexual (either male or female) and bisexual flower types. Consequently, depending on the flower type said plants can develop pollen or an ovule even in the same or in different flowers. The immature inflorescence thus represents the developmental precursor of the inflorescence comprising developing meristematic cells. Currently, it is common in the literature to perform plant regeneration starting from immature inflorescences *ex vivo*/*ex planta* by establishing *in vitro* callus cultures thereof (see e.g. Praveena and Giri, Physiol. Mol. Biol. Plants, 2012).

The method according to this first aspect thus allows the functional integration of a genetic construct of interest into the at least one meristematic cell of the meristematic tissue of the immature inflorescence directly *in planta* and, in one embodiment, allows the further *in vivo in planta cultivation* of a transformed cell or tissue.

The term "functional integration" or "functionally integrated" as used herein refers to the integration of a genetic construct of interest into at least one meristematic cell of a meristematic tissue which allows the transcription and/or translation and/or the catalytic activity and/or binding activity, including the binding of a nucleic acid molecule to another nucleic acid molecule, including DNA or RNA, or the binding of a protein to a target structure within the at least one meristematic cell. Where pertinent, the functional integration takes place in a certain cellular compartment of the at least one meristematic cell, including the nucleus, the cytosol, the mitochondrium, the chloroplast, the vacuole, the membrane, the cell wall and the like. Consequently, the term "functional integration" - in contrast to the term "stable integration" detailed above - implies that the genetic construct of interest is introduced into the at least one meristematic cell by any means of transformation, transfection or transduction by biological means, including *Agrobacterium* transformation, or physical means, including particle bombardment, as well as the subsequent step, wherein the genetic construct exerts its effect within or onto the at least one meristematic cell in which it was introduced. Depending on the nature of the genetic construct to be introduced said effect naturally can vary and including, alone or in combination, *inter alia,* the transcription of a DNA encoded by the genetic construct to a ribonucleic acid, the translation of an RNA to an amino acid sequence, the activity of an RNA molecule within a cell, comprising the activity of a guide RNA, or an miRNA or an siRNA for use in RNA interference, and/or a binding activity, including the binding of a nucleic acid molecule to another nucleic acid molecule, including DNA or RNA, or the binding of a protein to a target structure within the at least one meristematic cell, or including the integration of a sequence delivered via a vector or a genetic construct, either transiently or in a stable way. Said effect can also comprise the catalytic activity of an amino acid sequence representing an enzyme or a catalytically active portion thereof within the at least one meristematic cell and the like. Said effect achieved after functional integration of the genetic construct according to the present disclosure can depend on the presence of regulatory sequences or localization sequences which are comprised by the genetic construct of interest as it is known to the person skilled in the art. As the present invention comprises both embodiments directed to the stable integration of a genetic construct of interest as well as the transient introduction of a genetic construct of interest into at least one meristematic cell, the effect achieved by the functional integration of the genetic construct can further vary depending on the mode of introduction being either stable or transient. Stable integration, as detailed above, relies on the integration of the genetic construct into the genome of the at least one meristematic cell, including the nuclear genome as well as the genome of other plant compartments, whereas the transient introduction implies that the functional integration means the introduction of the genetic construct of interest into the at least one meristematic cell followed by its transcription and/or translation and/or catalytic activity and/or binding activity in the cell without the need of a stable integration into the genome of the meristematic cell.

In one embodiment according to this aspect the nucleic acid target sequence, where the functional integration of the genetic construct of interest takes place is the nuclear genomic DNA. In another embodiment according to this aspect the nucleic acid target sequence into which the functional integration of the genetic construct of interest is mediated is a mitochondrial or a plastid DNA, wherein the genetic construct comprises a localization sequence wherein this sequence mediates the localization of the genetic construct in the respective plant compartment of interest, for example, a mitochondrium or a chloroplast. In yet another embodiment, the functional integration refers to the delivery of a genetic construct of interest into at least one meristematic cell, for example into the cytosol or another plant compartment, without that a stable integration takes place.

In one embodiment according to the above and according to the further aspects of the present invention, the transformation is performed *in vitro* culture free.

In another embodiment, transformation *in planta,* transformed plant cell, tissue, organ or material is explanted and further cultivated *in vitro.*

As detailed above, the methods according to the present invention provide the advantage that both the transformation and the further development of a transformed at least one meristematic cell can take place *in planta* obviating the need for cumbersome *in vitro* cultivation steps for the regeneration of a plant or plant material therefrom. In certain embodiments, it might, however, be suitable to explant or dissect a plant cell, tissue, organ or material for further cultivation, screening or testing depending on the specific needs. Several methods for the *in vitro* cultivation of a plant cell, tissue, organ or material are available to the skilled person.

In another embodiment, the functional integration of the genetic construct of interest into the at least one meristematic cell of the meristematic tissue of the immature inflorescence of step (iv) of the above aspect is performed as (a) a stable integration so that the integrated genetic construct of interest, or a part thereof, is heritable to a progeny, preferably whereby a transformed gamete of the pollen or of the ovule is generated from the at least one transformed meristematic cell and the transformed gamete of the pollen or of the ovule is fused to a zygote from which the progeny comprising the genetic construct of interest is developed; as transgene, or (b) a transient introduction allowing a targeted genetic manipulation of the at least one meristematic cell of the meristematic tissue of the immature inflorescence through the genetic construct of interest or products thereof, wherein the targeted genetic manipulation but not the genetic construct of interest, or a part thereof, is heritable to a progeny, preferably whereby a genetic manipulated gamete of the pollen or of the ovule is generated from the transformed at least one meristematic cell and the genetic manipulated gamete of the pollen or of the ovule is fused to a zygote from which the progeny comprising the targeted genetic manipulation is developed.

As detailed above, under "Definitions", methods for transforming or manufacturing a plant, plant cell, tissue, organ or material can rely on either the stable integration of a genetic construct or on the transient introduction of a genetic construct or a product thereof according to the present disclosure. The choice of the method, either stable or transient, predominantly is dictated by the effect which is intended to be achieved. Common genetic constructs suitable to be transformed according to the present invention can *inter alia* be selected from the group consisting of at least one of a viral vector, a CRISPR/Cas encoding construct, an RNAi encoding construct, an miRNA encoding construct, a construct comprising a resistance gene, a construct comprising a fluorescent marker gene, a genetic construct designed to introduce a targeted knock-out of a plant gene or region of interest, a genetic construct designed to introduce a targeted knock-in of a transgene of interest at a plant gene or region of interest, the introduction of a genetic construct mediating a homology-directed repair at a region, where a DNA double-strand break occurred, and a combination thereof.

A stable integration might thus be desirable, where a transgenic plant carrying a desired construct of interest, or a part thereof, is stably inserted and the inserted construct or part thereof is inherited to the progeny of a plant cell of interest initially transformed. Said stable integration can take place into any genomic region of the plant, including the nuclear genome as well as the extranuclear genome, including the genome of plastids of a plant cell.

A transient introduction might be desirable, in case a certain effect, e.g. a RNA mediated silencing effect, a targeted manipulation, comprising a knock-in or a knock-out, is desired by the introduction of a genetic construct of interest, or a part thereof, but the construct *per se* should not be inherited to a progeny of the cell initially transformed.

In another embodiment of the above aspect, the method comprises an additional step (v) comprising determining the functional integration of the genetic construct of interest.

In one embodiment, said determination can take place *in situ,* i.e. directly in plant by analyzing a transformed at least one meristematic cell or tissue of interest, or a progeny thereof.

In another embodiment, said determination can take place *in vitro* by explanting or dissecting a transformed at least one meristematic cell or tissue, either as a whole, or a part thereof.

Methods for analyzing a functional integration are known to the person skilled in the art and comprise, but are not limited to polymerase chain reaction (PCR),including inter alia real time quantitative PCR, multiplex PCR, RT-PCR, nested PCR, analytical PCR and the like, microscopy, including bright and dark field microscopy, dispersion staining, phase contrast, fluorescence, confocal, differential interference contrast, deconvolution, electron microscopy, UV microscopy, IR microscopy, scanning probe microscopy, the analysis of plant or plant cell metabolites, RNA analysis, proteome analysis, functional assays for determining a functional integration, e.g. of a marker gene or a transgene of interest, or of a knock-out, Southern-Blot analysis, sequencing, including next generation sequencing, including deep sequencing or multiplex sequencing and the like, and combinations thereof.

In yet another embodiment of the above aspect according to the present invention, the introduction of the genetic construct of interest according to step (iv) of the above aspect is conducted using a means selected from the group consisting of a device suitable for particle bombardment, including a gene gun, including a hand-held gene gun (e.g. Helios® Gene Gun System, BIO-RAD) or a stationary gene gun, transformation, including transformation using *Agrobacterium spp.* or using a viral vector, microinjection, electroporation, whisker technology, including silicon carbide whisker technology, and transfection, or a combination thereof.

Several methods are known to the skilled person suitable for plant cell transformation. As a prerequisite, said transformation methods all require a plant, plant cell, tissue organ or material to be transformed in a readily accessible state and in a condition so that the respective sample, including at least one cell, is viable. The present invention thus provides a basic method for allowing accessibility of a meristematic cell or tissue of a plant as target structure in a viable state to allow the further manipulation or analysis of the thus exposed target structure *in planta.*

There is provided a further embodiment, wherein step (ii) of the above aspect comprises the production of an artificially introduced window in the region, where the meristematic tissue of the immature inflorescence is located, preferably in the shoot axis, in particular in the axil, halm, culm or stem of the plant. As it is known to the skilled person in the field of plant biology, the morphology of different plant species can significantly vary and thus the nomenclature for the shoot axis will vary depending on the plant to be characterized.

The term "artificially introduced window" as used herein refers to a purposively introduced lesion being introduced *in planta,* this means into a growing plant, plant material or seedling, wherein the artificially introduced window is positioned in a region, where meristematic tissue of the immature inflorescence, comprising at least one meristematic cell, is located. As detailed above and as known to the skilled person, meristematic regions or meristematic cells of a developing plant are often surrounded by further tissues which keep the developing meristematic tissue in a protected state. The dimensions of the artificially introduced window naturally can vary depending on the plant, into which the window is introduced. The dimensions (length, width and height) are in a size to fulfill the following conditions: (i) exposition of at least one cell of a meristematic tissue of the immature inflorescence so that said region is preferably macroscopically detectable; and (ii) (for full *in planta* approaches) the window may be such that the plant is still viable after introduction of the window and can further develop and proceed through further developmental stages, i.e. the window preferably may not remove more than necessary to expose the meristematic tissue comprising at least one meristematic cell. In other embodiments, where the artificially introduced window is created to expose a meristematic tissue of interest to make it accessible for the further explantation/dissection, the window can have greater dimensions to ease the dissection of the material. In this embodiment it is not necessary to take into consideration the further fate of the plant. It is, however, mandatory not to destroy or damage the target structure of interest, i.e. at least one meristematic cell of a meristematic tissue, which is to be cultivated or analyzed further *in vitro.* The opening of the window can be achieved using physical or mechanical means.

To achieve a suitable artificial window for a selected target plant, first, the developmental stage of the plant is checked by counting the number of leafs. Depending on the genotype, the introduction of the artificial window can be started between the 3 -10 leaf stage, preferably between the 6- and 10-leaf stage. By touching the stem of the plant, the area where the real stem is ending, i.e. the position where the tassel will be located can be determined. Next, a window, preferably with rectangular shape, but not restricted thereto, is cut into the stem with the help of a scalpel or any another mechanical means for opening the stem of the plant of interest exactly at the location where the tassel is likely to be located at. The different leaf sheets are then removed layer by layer until the immature tassel is reached (see e.g. **Fig. 5**)**.** In the last step, preferably forceps are used to remove the plant material surrounding the tassel to be exposed. As a general rule, the size of the window has to be as small as possible for not disrupting the further development of the plant. The minimum size of the artificial window thus is in the range of approximately 0.5 x 0.5 cm. Depending on the plant, the window can also be smaller, e.g. approximately 0.25 x 0.25 cm or even below. A very small window size might possibly complicate the further handling. Using specific equipment, including micromanipulation tools, including *inter alia* optical tweezers or laser microbeam-assisted tools, the creation of even very small windows can be created.

In another embodiment, the artificial window can have dimensions from around 1 cm x 1 cm, or, if a rectangular window is preferred, approximately 1 cm x 0.5 cm, approximately 1.5 x 0.5 cm, approximately 1.5 x 1 cm or approximately 2 cm x 1 cm or any dimensions in between and it can even have larger dimensions depending on the stem architecture of the plant of interest. In principle, there is no maximum size as long as the natural development of the plant is not impeded. There is also no limitation to the geometry of the window, i.e. round or oval windows or the other geometries can be produced as well. Then, a window can have dimensions ranging from a radius of about 0.1 to 2 cm, preferably from a radius of about 0.2 to 1.5 cm, more preferably from a radius of about 0.5 to 1 cm. The specific dimensions will depend on the plant to be windowed and can be determined using practical criteria of handling the respective plant.

In embodiments, where the further cultivation steps are intended to be performed *in vitro* the window can also be larger, as it is not necessary that the plant can further proliferate after exposition and removal of the meristematic structure, preferably an immature inflorescence, after the window opening.

Preferably, a vertical cut is made from the top of the artificial window towards the leaf whorl in order to help the tassel to grow vertically. After cutting the window, the window is preferably covered back again by one or two cut leaf lids. To prevent the tassel from drying, a piece of wetted material, e.g. cotton (using water or water and a fungicide) can be inserted between the tassel and the lid. The whole artificial window including the lid can then be wrapped with a tissue paper and secured with a metallic twist tie.

In embodiments, where particle bombardment as physical means of transformation is used, the tissue paper, the leaf lid and the cotton are removed and the target structure of interest can then be bombarded before the cotton, the lid and the tissue are again placed back onto the artificial window. To assure a natural development of the exposed tassel tissue, the growth of the tassel is checked daily after transformation. In certain embodiments, it might be suitable to remove that or necrotic tissue to assist the tassel in growing vertically by opening the leaf sheets. In embodiments, where a tissue paper is used, said paper can be removed after around 3 - 5 days after transformation, including transformation via particle bombardment.

To be able to manipulate at least one meristematic cell of a meristematic tissue, preferably of the immature inflorescence of a plant, it is thus required to expose said meristematic tissue comprising at least one meristematic cell to be manipulated or modified. The artificially introduced window according to the present invention thus has to be made in the region where the target structure of interest is located. Furthermore, in one embodiment the artificially introduced window should be suitable to keep the at least one cell of the meristematic tissue in an exposed state to monitor the further development thereof, if desired, which is preferable for performing intermediate analysis to determine whether a transient introduction or a stable integration according to some embodiments according to the present invention have occurred.

In addition, the artificially introduced window may not destruct the whole plant organism or disturb the further development of the plant, as this would obviate an *in planta* manufacturing approach according to the present disclosure, wherein the living plant comprising the transformed at least one meristematic cell is further cultivated *in planta* until a desired developmental stage of the transformed material is achieved. As it is known to the skilled person, the region where an artificially introduced window has to be purposively created to perform the methods according to the present invention can vary depending on the target plant of interest, as the region, where the meristematic tissue of the immature inflorescence is located varies from plant species to plant species. In embodiments, where the further development of the transformed at least one meristematic cell is not proceeded through in an *in vitro* culture free way, the "artificially introduced window" can have huger dimensions to fully expose a target region of interest, comprising at least one meristematic cell. This is due to the fact that the integrity of the living plant is not a prerequisite in case the further cultivation steps of the transformed material will be conducted *in vitro,* e.g. via callus regeneration and the like.

In yet another embodiment of this aspect according to the present invention the plant or the plant material is or is part of a monocotyledonous (monocot) plant, or is or is part of a dicotyledonous (dicot) plant.

Monocot and dicot plants, both belonging to the flowering plants or angiosperms, differ mainly in that monocot plants have only one seed leaf (cotyledon) within the seed the shoot is evolving from and dicots have two seed leaves or cotyledons the shoot is evolving from. Both dicot and monocot plants, however, proceed through common stages during plant development for yielding the fully differentiated flowers. Therefore, the methods according to the present invention can be applied for any kind of monocot or dicot plant having a plant architecture, i.e. a three-dimensional organization of the plant body, comprising at least one a meristematic cell of a meristematic tissue of an immature inflorescence and, optionally wherein the plant architecture allows the production of an artificially introduced window in the region, where the meristematic tissue of the immature inflorescence is located.

In a further embodiment according to the above aspect according to the present invention the plant or the plant material is or is part of a plant from a species selected from the group consisting of *Hordeum vulgare, Hordeum bulbusom, Sorghum bicolor, Saccharum officinarium, Zea mays, Setaria italica, Oryza minuta, Oriza sativa, Oryza australiensis, Oryza alta, Triticum aestivum, Secale cereale, Malus domestica, Brachypodium distachyon, Hordeum marinum, Aegilops tauschii, Daucus glochidiatus, Beta vulgaris, Daucus pusillus, Daucus muricatus, Daucus carota, Eucalyptus grandis, Nicotiana sylvestris, Nicotiana tomentosiformis, Nicotiana tabacum, Solanum lycopersicum, Solanum tuberosum, Coffea canephora, Vitis vinifera, Erythrante guttata, Genlisea aurea, Cucumis sativus, Morus notabilis, Arabidopsis arenosa, Arabidopsis lyrata, Arabidopsis thaliana, Crucihimalaya himalaica, Crucihimalaya wallichii, Cardamine flexuosa, Lepidium virginicum, Capsella bursa pastoris, Olmarabidopsis pumila, Arabis hirsute, Brassica napus, Brassica oeleracia, Brassica rapa, Raphanus sativus, Brassica juncea, Brassica nigra, Eruca vesicaria subsp. sativa, Citrus sinensis, Jatropha curcas,Populus trichocarpa, Medicago truncatula, Cicer yamashitae, Cicer bijugum, Cicer arietinum, Cicer reticulatum, Cicer judaicum, Cajanus cajanifolius, Cajanus scarabaeoides, Phaseolus vulgaris, Glycine max, Gossypium sp., Astragalus sinicus, Lotus japonicas, Torenia fournieri, Allium cepa, Allium fistulosum, Allium sativum,* and *Allium tuberosum,* or any variety belonging to one of the aforementioned plant species.

In another embodiment according to the first aspect of the present invention, the immature inflorescence is a male inflorescence (tassel) of a *Zea mays* plant.

The overall architecture of the tassel of a maize plant is depicted in **Fig. 1****.** The tassel, or male inflorescence, in maize is highly branched with long lateral branches at the base of the main spike. Short branches, called spikelet pairs, are produced by the main axis and the long branches. Each spikelet is composed of two reduced leaf-like glumes enclosing two florets (see **Fig. 1**). Each floret consists of two reduced leaves called the lemma and palea, two lodicules (the remnants of the petals) (Ambrose et al., 2000), three stamens and a tricarpellate gynoecium. In the tassel, the gynoecium aborts resulting in the formation of male florets (Cheng et al., 1983; Irish, 1996). The female inflorescence (the ear shoot) forms from an axillary meristem located in the axil of a leaf five to six nodes below the tassel. The ear does not produce long lateral branches but does produce paired spikelets with paired florets like the tassel. Subsequently, the lower floret and the stamens abort resulting in the formation of single female florets (Cheng et al., 1983; Irish, 1996). Three types of axillary meristems are involved in producing this complex inflorescence (see McSteen et al., 2000 and McSteen and Hake, 2001). The first axillary meristems produced by the inflorescence meristem are the branch meristems. Branch meristems at the base of the tassel produce the long lateral branches while later arising branch meristems (also called spikelet pair primordia) produce two spikelet meristems. Each spikelet meristem forms two glumes and two floral meristems. Subsequently, each floral meristem gives rise to the floral organs. Targeting of meristematic cells of said meristematic tissues is thus favourable to generate a targeted genetic manipulation in the maize male inflorescence. Based on the methods according to the present invention, in one embodiment there is thus provided an approach for directly targeting at least one meristematic cell of a meristematic tissue of the developing tassel of a maize plant *in planta* which allows the targeted manipulation of this tissue type finally resulting in the reproductive organs and cells. This approach thus paves the way for an *in vitro* culture free genetic manipulation of this important crop plant within one generation, as the final result of a targeted genetic manipulation can already be obtained in the plant to be manipulated without the need for further cultivation or crossing. The methods according to the present invention are thus suitable for producing chimeric plants or later on transgenic or genetically manipulated plants that will have transformed reproductive cells, tissues or organs.

In yet another embodiment of the present invention, the introduction of the genetic construct of interest as defined in step (iv) according to the above aspect is performed at a developmental stage, either during the stamen initiation process or before spikelets are formed on the male inflorescence.

Given the plant development of monocot and dicot plants, the methods of the present invention specifically targeting at least one meristematic cell of a plant inflorescence as long as meristematic cells are present. For certain approaches, it might be favorable to do the transformation in an earlier developmental state of the inflorescence development to (i) potentially affect a larger cell population of the progenies of the at least one meristematic cell to be transformed and/or (ii) depending on the type of transformation chosen to have less cell layers (glumes, lemma or palea or the epidermis of the anther) surrounding the target tissue or cell. In several dicot plants, where floral meristems can be produced directly from the inflorescence meristem without proceeding through branch and spikelet meristems, it might likewise be of interest to target at least one cell of the inflorescence meristem during an early developmental stage to place the genetic manipulation of interest into as much as possible meristematic cells or progeny thereof.

In a second aspect according to the present invention there is provided a method for manufacturing a transgenic plant comprising the following steps: (i) providing an *in planta* transformed plant or plant material transformed by a method according to the first aspect of the present invention; (ii) cultivating the plant or plant material under suitable conditions until the developmental stage of maturity of the inflorescence is achieved, (iii) allowing the transformed gamete of the pollen or of the ovule generated from the transformed at least one meristematic cell to fuse to a zygote comprising the genetic construct of interest as transgene; and (iv) developing the transgenic plant from the zygote.

In a third aspect according to the present invention there is provided a method for manufacturing a genetically manipulated plant comprising the following steps: (i) providing a *in planta* transformed plant or plant material transformed by a method according the first aspect of the present invention detailed above; (ii) cultivating the plant or plant material under suitable conditions until the developmental stage of maturity of the inflorescence is achieved; (iii) allowing the genetically manipulated gamete of the pollen or of the ovule generated from the transformed at least one meristematic cell to fuse to a zygote comprising the targeted genetic manipulation; and (iv) developing the genetically manipulated plant from the zygote.

In one embodiment according to the second or the third aspect of the present invention, the step of cultivation of the plant or plant material under suitable conditions until the developmental stage of maturity of the inflorescence is achieved is performed *in vitro* culture free.

In another embodiment according to the second or the third aspect of the present invention, the step of cultivation of the plant or plant material under suitable conditions until the developmental stage of maturity of the inflorescence is achieved is performed *in vitro.* Several protocols are available to the skilled person for producing germinable and viable pollen from *in vitro* cultured maize tassels as disclosed, for example in Pareddy DR et al. (1992) Maturation of maize pollen in vitro. Plant Cell Rep 11 (10):535-539. doi:10.1007/BF00236273, Stapleton AE et al. (1992) Immature maize spikelets develop and produce pollen in culture. Plant Cell Rep 11 (5-6):248-252 or Pareddy DR et al. (1989) Production of normal, germinable and viable pollen from in vitro-cultured maize tassels. Theor Appl Genet 77 (4):521-526. Those protocols are *inter alia* based on excision of the tassel, surface sterilization and culture in a media with kinetin to promote tassel growth and maturation. After the spikelets are formed, they a continuous harvest of anthers can be performed. After extrusion, anthers will be desiccated until the pollen comes out. Alternatively, anthers can be dissected and the pollen is shed in liquid medium that is subsequently used to pollinate ears.

"Maturity of the inflorescence" as used herein refers to the state, when the immature inflorescence of a plant comprising at least one meristematic cell has reached a developmental stage, when a mature inflorescence, i.e. a staminate inflorescence (male) or a pistillate inflorescence (female), is achieved and thus a gamete of the pollen (male) or of the ovule (female) or both is present. Said stage of the reproductive phase of a plant is especially important, as the obtained plant material can directly be used for pollination of a further plant or for fertilization with the pollen of another plant.

"Suitable conditions" or "suitable reaction conditions" as referred to herein in the context of the transformation or manufacturing methods according to the present disclosure refer to conditions, which allow both, the growth and development of the plant being transformed or manufactured and the conditions necessary for achieving either stable integration or transient introduction of a genetic construct of interest in the at least one meristematic cell of interest. Conditions to promote plant growth and development, including *inter alia* temperature, light, water, oxygen, mineral nutrients and soil support, which can vary for different plant species/cultivars can be readily determined by the skilled person in knowledge of the disclosure provided herein. The further suitable conditions to achieve stable integration or transient introduction of a genetic construct of interest depend on the transformation method selected for introduction of the genetic construct, the developmental age of the plant material or plant cell to be transformed and the genetic construct of interest to be introduced. Said suitable conditions can be defined by the skilled person in light of the present disclosure defining the suitable conditions for the methods in combination with exemplary genetic constructs as disclosed and claimed herein.

In one embodiment, where all steps according to the methods of the present invention proceed *in vitro* culture free, the term "suitable conditions" further can imply that the transformed at least one meristematic cell of the immature inflorescence is kept in a continuing accessible state so that the resulting progeny of the transformed at least one meristematic cell can be directly obtained from the viable plant in a viable state. The term thus implies that, when necessary, an artificially introduced window is monitored and optionally enlarged, as the plant organism by natural phenomena driven by the wound healing capacity of a plant organism might regenerate the plant tissues surrounding the developing meristematic tissue of interest.

In still another aspect according to the present invention there is provided a plant manufactured by the manufacturing methods of the second or third aspect of the present invention, or plant cells, a plant material, or derivatives or a progeny thereof.

In one embodiment of this aspect, the manufactured plant is a monocot plant, in another embodiment the plant is a dicot plant.

In a further embodiment according to this aspect according to the present invention the plant or the plant material is or is part of a plant from a species selected from the group consisting of *Hordeum vulgare, Hordeum bulbusom, Sorghum bicolor, Saccharum officinarium, Zea mays, Setaria italica, Oryza minuta, Oriza sativa, Oryza australiensis, Oryza alta, Triticum aestivum, Secale cereale, Malus domestica, Brachypodium distachyon, Hordeum marinum, Aegilops tauschii, Daucus glochidiatus, Beta vulgaris, Daucus pusillus, Daucus muricatus, Daucus carota, Eucalyptus grandis, Nicotiana sylvestris, Nicotiana tomentosiformis, Nicotiana tabacum, Solanum lycopersicum, Solanum tuberosum, Coffea canephora, Vitis vinifera, Erythrante guttata, Genlisea aurea, Cucumis sativus, Morus notabilis, Arabidopsis arenosa, Arabidopsis lyrata, Arabidopsis thaliana, Crucihimalaya himalaica, Crucihimalaya wallichii, Cardamine flexuosa, Lepidium virginicum, Capsella bursa pastoris, Olmarabidopsis pumila, Arabis hirsute, Brassica napus, Brassica oeleracia, Brassica rapa, Raphanus sativus, Brassica juncea, Brassica nigra, Eruca vesicaria subsp. sativa, Citrus sinensis, Jatropha curcas,Populus trichocarpa, Medicago truncatula, Cicer yamashitae, Cicer bijugum, Cicer arietinum, Cicer reticulatum, Cicer judaicum, Cajanus cajanifolius, Cajanus scarabaeoides, Phaseolus vulgaris, Glycine max, Gossypium sp., Astragalus sinicus, Lotus japonicas, Torenia fournieri, Allium cepa, Allium fistulosum, Allium sativum,* and *Allium tuberosum,* or any variety belonging to one of the aforementioned plant species.

In yet another aspect according to the present invention there is provided a plant, preferably a *Zea mays* plant, comprising an artificially introduced window in a region, where a meristematic tissue of an immature inflorescence is located, preferably in the axil at the culm of the plant, preferably wherein the plant is a plant *in planta* transformed by a method according to the first aspect according to the present invention.

In one embodiment, the plant provided according to this aspect is a viable plant. In another aspect the plant is a harvested plant, or a plant cell, tissue, organ or a plant material thereof.

In a further aspect according to the present invention there is provided the use of a plant comprising an artificially introduced window in a region, where a meristematic tissue of an immature inflorescence is located, preferably in the axil at the culm of the plant, preferably wherein the plant is a plant *in planta* transformed by a method according to the first aspect according to the present invention, or of a plant *in planta* transformed by a method according to the first aspect according to the present invention, for the manufacture of a transgenic or genetically manipulated plant, plant cell or plant material.

As evident from the various aspects and embodiments according to the present invention, a plant having an artificially introduced window in a region, where a meristematic tissue of an immature inflorescence is located, preferably in the shoot axis, in particular in the axil, halm, culm or stem of the plant, is specifically suitable for a variety of approaches for manipulating the genetic material of at least one meristematic cell of said meristematic tissue, for performing the functional integration of a genetic construct of interest, or for analytical or testing purposes for studying the natural development of the plant. In the natural environment of certain plants, the immature inflorescence meristem is well protected by surrounding cell layers and tissues during early developmental stages. Therefore the provision of a plant having an artificially introduced window exposing the immature inflorescence is useful for the plant transformation and manufacturing methods according to the present invention as well as for further analytical and screening purposes.

The skilled person in the field of plant biotechnology is well aware of the fact that plant organogenesis proceeds differently in various plants, especially when comparing monocot and dicot plants. Therefore, the localization of meristematic cells and tissues which build up the inflorescence meristem or which can develop into the inflorescence meristem is different into the various plant species. Furthermore, the development of floral meristems naturally differs from plant species to plant species. Thus, the floral meristem can either directly develop from the inflorescence meristem, like in dicots like *Arabidopsis* or *Antirrhinum,* or the floral meristem can develop from the inflorescence meristem via branch and spikelet meristems, like for maize (see **Fig. 1**)**.** The person skilled in the art having knowledge about the disclosure of the present invention and the detailed Examples for different plant species disclosed herein can thus transfer the methods disclosed herein to a variety of target plants of interest.

The present invention is further described with reference to the following non-limiting examples.

### Examples

The present invention is further illustrated by the following non limiting examples.

### Example 1: Maize tassel development

In corn development, the apical meristem differentiates into tassel primordia in the V6 stage ("Vegetative stage 6" or when the plant has 6 leaves) or 25-30 days after germination (Cheng et al. 1983; Abendroth et al. 2011). Therefore, it was tried to target the cells of those immature tassels that will further develop in the anther tissue that will produce pollen. One set of experiments focused on maize plant, namely A188 plants. Already 1 month old plants have developing tassels instead of the classical apical meristems in these plants see **(****Fig. 3** **and** **4****).** It was observed that the growth of the immature tassel is very fast, going from less than 1 cm to more than 8 in 11 days **(****Fig. 4****).** We also looked at the developmental stage of the spikelets and decided that the target age will be between 34 and 37 days after sowing, when the tassel is easy to be detected and exposed but the spikelets are not formed **(****Fig. 4****).** This is an important parameter, in case the immature inflorescence is intended to be transformed via a physical method like particle bombardment, as the more cell layers (glumes, lemma or palea and the epidermis of the anther) the particles have to go through to reach pollen producing tissues, the better it is to choose an early developmental stage. For biological transformation approaches, including *Agrobacterium* transformation, e.g. using viral vectors, there is a greater flexibility in the time range for conducting transformation, as the biological cargo will more easily reach the meristematic target cells of interest without disrupting the surrounding cells and tissue.

### Example 2: Exposing the immature inflorescence of a growing maize plant

To facilitate the genetic manipulation or the introduction of a genetic construct into the maturing tassel tissue, several methods were analyzed. It was found that a window could artificially be introduced into the region, where the meristematic tissue of the immature inflorescence is located. For maize, this region is located near the axil in the shoot axis, the culm, of the plant. An exemplary plant comprising an artificially introduced window is shown in **Fig. 5****.** The tassel is pushed upwards very fast during plant development. When the tassel is exposed through a window, the natural pathway to grow upwards is disrupted. Sometimes the tassel grows out of the plant or other is trapped between the growing leaf mass, making the plants look abnormal in comparison to a non treated plant (see e.g. **Fig. 7** **(D to E)**), which, however, did not disturb the overall plant and tassel development. For a maize plant, it is thus recommendable to perform a vertical cut from the existing artificially introduced window to the top of the plant to allow the growing tassel to move upwards and mature.

It was observed that in most of the "windowed" plants the tassels are producing pollen, but not all of them develop ears. Usually those plants mature several weeks before a normal A188 maize plant would and they are in general shorter and weaker plants, which however, did not influence the further transformation and analytical experiments as conducted with the exposed material, neither *in planta* nor *in vitro.*

### Example 3: Transient transformation of exposed immature tassel with particle bombardment in vitro

One of the questions to be answered by this technology was if the exposed immature tassel tissue is amenable for transformation, especially *in planta,* but also *ex vivo.* For that, several experiments were conducted in which the immature tassel was separated from the plant and bombarded in the lab using the bench gene-gun PDS-1000/He (BIO-RAD). Particle bombardment turned out to be a suitable method for all experiments conducted with immature meristematic tissues and isolated cells from several plants including maize, barley and wheat varieties for both *in vitro* as well as *in planta* bombardment. Experiments with several test constructs of dsDNA, RNA and proteins as well as virus particles were used in the different settings. During this series of experiments it turned out that the parameters for bombardment are critical to influence, whether a transient or stable expression of a dsDNA construct will be achieved. More heavy bombardment was observed to increase the rate of transient introduction and also can cause a damage of the material bombarded. It is thus necessary to establish the best mode of bombardment individually for each target tissue to be transformed.

Usually, for plant transformation through bombardment, the gene gun known as PDS-1000/He (BIO-RAD) is used, but any comparable device can be used for the presented protocols. The Helios gene-gun is a bench apparatus that relies on helium pressure to discharge the particles to the plant tissue under vacuum conditions. The explants that are bombarded for stable plant transformation purposes necessarily undergo *in vitro* culture, this is why the explants are usually bombarded on an *in vitro* culture plate. The Helios gene gun was designed initially to do *in situ* transformation of animal cells and tissues. The gun can operate without vacuum conditions and at a lower shot pressure, minimizing cell damage (Finer et al. 2000). The use of the Helios gene gun in plant transformation has been limited to transient expression experiments *in vitro* and no assay could ever be performed *in vitro* culture free.

The tassel in the present experiments as shown in **Fig. 6** is able to transiently express the delivered construct (here: containing a commercially available red fluorescent gene expression cassette). This expression can even be observed in the immature spikelets, what it is an indication that we could be targeting tissue that will produce pollen **(****Fig. 6****).**

For this experiment, an immature tassel from a V7-V8 plant was detached and placed into on a petri dish with MS media (MS salts + vitamins, 20 g/L sucrose, 3 g/L phytagel, pH 5.7). Notably, V7 or V8 stage implies that the collar of the 7^{th} or 8^{th} leaf is visible, respectively. The tassel was bombarded once using a red fluorescent protein expressing plasmid coated using standard calcium chloride/spermidine coating on 0,6 µm gold particles. The bombardment was done at 1100 psi, 3^{rd} shelve from the top of the gene gun. After bombardment the tassel was placed in darkness and the next day, the transient expression was checked under a fluorescence microscope.

### Example 4: Stable transformation of exposed immature tassel with particle bombardment in planta

In the next step the hand held gun Helios was used for bombardment *in planta* of the exposed immature tassel. Pressures between 100 and 200 psi and from 1 to 6 shots per tassel were used. The distance to the target is the one of the Barrel liner, around 3 cm. It was observed that higher pressures and higher number of bombardments damage the tassel branches. However, transient expression in tassel samples taken 1 day after bombardment **(****Fig. 7****)** could be observed. In most of the cases, the bombarded tassels have been able to develop and shed pollen. As tassel tissues like anthers and dry pollen have a strong autofluorescence, a fluorescence detection means can be accomplished and confirmed by further molecular methods, like PCR, including enrichment PCR, or sequencing, including next generation sequencing, including *inter alia,* deep sequencing, or multiplex sequencing and the like. Bombarded tassels were regularly observed several days after bombardment. Red fluorescent spikelets and glume cells could be observed **(****Fig. 8****)** from 7 to 28 days after bombardment, what was indicative of stable transformation events in those bombarded tassels. These results were confirmed by PCR, RT-PCR, sequencing, including deep sequencing or other next generation sequencing approaches, or Southern blot analysis.

As usual set up for analyzing a stable integration event in different target plants was as follows: First, DNA and/or RNA were extracted of different material including tassel, anther or pollen tissue/cells transformed with different constructs encoding a red fluorescent protein. In sum, 89 samples were analyzed via quantitative PCR (qPCR). From the above samples, 20 samples showing a clear, i.e. a very intense, red fluorescent signal were selected. From those 20 samples cDNA was generated including controls without reverse transcriptase to exclude that the later results are not associated with undigested DNA. Out of the 20 samples with positive DNA signal used for the transcription measurement, 6 samples (6.7%) showed a clear transcription and 3 a potential transcription (at the border of what could be clearly measured). In **Table 1** below the results for this experiment are listed. Especially in tassel branches very clear signals could be obtained. Also in pollen clear transcription signals have been observed. A further round of experiments changing the parameters for bombardment was done to increase the frequency of stable integration. It was observed that a lower shot pressure and/or choosing a different time point of bombardment also contributed to achieving a stable integration detectable for anther tissue (data not shown).

**Table 1: Results: Stable integration in 89 tested samples**

| Type of material | DNA signal | Transcription |
|---|---|---|
| Tassel Branch | 18 of 75 | 7 of 18 |
| Immature Tassel | 1 of 6 | 1 of 1 |
| Anthers | 0 of 3 | |
| Pollen | 1 of 5 | 1 of 1 |

### Example 5: Transformation of exposed immature tassel tissue

As detailed above, a variety of physical/mechanical as well as biological means for transforming plant cells, tissues, organs or whole plants or parts thereof have been described for introducing genetic material into a plant or plant target structure. After having exposed and thus obtained a tassel tissue according to the disclosure of the present invention, the following methods can be applied to transform this tissue.

Concerning biological means, the tassel tissue or cells thereof can be transformed with *Agrobacterium,* including *Agrobacterium tumefaciens* or *Agrobacterium rhizogenes* mediated transformation. This kind of transformation is well known to the person having skill in the art (see e.g. Jones, H.D. et al., "Review of methodologies and a protocol for the Agrobacterium-mediated transformation of wheat", plant methods, 2005; or Frame, B.R. et al., "Agrobacterium tumefaciens-mediated transformation of maize embryos using a standard binary vector system", Plant, 2002). To this end, an *Agrobacterium* culture comprising a construct of interest is, for example, cultivated over night at 28°C in fluid Luria Broth medium containing a suitable antibiotic, 10 mM MES and 200 mM ACE. The next day, the over night cultured is centrifuged at 4.400 rpm for 15 min and the supernatant is discarded. The pellet is then again centrifuged for 15 min at 4.400 rpm for 2 min and the remaining supernatant is discarded. The pellet is resuspended (5 ml H₂O, 10 mM MES, 10 mM MgCl₂ + 20µM ACE). The optical density at 600 nm is adjusted to 1.5. The possibly diluted suspension can then be further used.

Another possibility for transforming immature tassel tissues via biological means is the use of viral vectors. Viral vectors have the advantage that they can be introduced either as DNA or as RNA and to a plant target structure of interest. Furthermore, viral vectors or plant viruses have the capability of spreading into different cells.

For the purpose of the present invention, virus particles, *in vitro* transcripts of viruses or *Agrobacteria* carrying a virus encoding T-DNA have been introduced into a plant target structure of interest via filtration (vacuum and non-vacuum). Alternative experiments were carried out using plant sap. To this end, either tobacco or spinach have been infected with the virus of interest to subsequently isolate said virus of interest from the plant sap for infecting another plant target structure, especially tassel tissues from different plants with the plant sap containing the virus.

Despite the biological means of transforming tassel structures of interest, further physical/mechanical means for transformation in addition to particle bombardment were tested.

One suitable method turned out to be microinjection. Microinjection can be used for any kind of meristematic structure tested, preferentially using a microscope with a micromanipulator. Due to the size of certain meristematic structure like tassel or ear meristems, microinjection can be conducted under microscope control or, in case where the target structures are large enough, without microscope assistance. The injection can be conducted, using a variety of methods for a variety of different target molecules to be introduced into a plant target structure of interest including double-stranded plasma DNA, linear double-stranded DNA, RNA and proteins as well as virus particles in liquid solution. These different molecules can be applied with the help of a micro- or nano-needles which assists in injecting the target molecules into the meristematic cell or structure of interest. The target molecules are first coated onto the needle which is then inserted into the meristematic cell or structure of interest.

A further development of this technology is the use of a combination of silicon carbide (SiC) whiskers (e.g. Silar® Silicon Carbide Whisker) and microinjection. To this end, double-stranded plasma DNA, linear double-stranded DNA, RNA, protein or virus particles are precipitated onto the silicion carbide whisker to be injected via a microinjection needle into the meristematic structure or cell of interest. This technique has the advantage that it is not only possible to transfect a single meristematic cell, but there is the possibility to penetrate different cells in parallel due to the spread of the whiskers. Furthermore, the cells get less destructed, as the needle does not have to penetrate into the cell and the whiskers are quite small in size.

### Example 6: Optimization of further parameters

Depending on the plant species chosen for transformation and depending on transformation methods, several parameters were adjusted to achieve an improved transformation efficiency. In the case of physical transformation methods, especially particle bombardment, a balance between DNA delivery efficiency and damage of the plant tissue has to be determined when a meristematic tissue of interest, either *in planta* or *in vitro* as an explants, is transformed. For particle bombardment the parameters tested were helium pressure, number of shots, amount of gold per shot, size of gold particles, amount of DNA per shot, distance to the target, positioning of the gene gun in respect to the tassel or the use of a protective metallic screen. Depending on the target structure to be transformed the shot pressure can be in the range of about 10 to 1500 psi, preferably in the range of about 10 to 1000 psi, more preferably in the range of about 10 to 500 psi, even more preferably in the range of about 50 to 400 psi and most preferably in the range of about 50 to 300 psi. Naturally, the shot pressure will vary depending on the target plant the material is to be bombarded as well as on the result to be achieved (transient versus stable integration of a construct). Preferably, the shot pressure is in the range from 100 to 200 psi. The particle size of the particles, e.g. gold or tungsten, chosen as carrier for particle bombardment can be in the range of about 0.4 to 2 µm as commercially available, yet other dimensions of particles are possible. The particles should not exceed a certain size as this would lead to a further destruction of plant material which could hamper the present protocol if fully conducted *in planta*/*in vivo..* Preferably, the particle size is in the range from about 0.6 to 1 µm. A certain degree of damage to the plant tissue to be transformed was well tolerated for *in planta* approaches and, especially for more mature tassels in maize, provided a better transformation efficiency without hampering the further development of the tassel.

Another factor to be determined is the best developmental age or stage of a plant at the time when transformation is performed. Generally, the more immature the tassel, the easier it will be to target cells that will differentiate in the anther tissue that will produce the pollen. As detailed in **Fig. 4****,** a series of experiments was performed for maize. It was observed that 34 day old plants have a very small tassel difficult to expose, this tassel is very sensitive to bombardment, being damaged very easily. However, hitting one cell in this immature tassel has more chances to become a tissue for pollen production. On the other hand, 37 day old plants were bombarded. In these plants, the main branch of the tassel has already differentiated spikelets. The target in this case are the more undifferentiated branches of the tassel located at the bottom. Those bigger tassels are more resistant to bombardment, but the particles have to go through more layers of cells to reach the sporogenic tissues. Using alternative transformation methods like *Agrobacterium* transformation of immature tassels or any other transformation method suitable for a plant cell, tissue or organ or a whole plant or material thereof can be applied.

2 approaches were followed in addition to particle bombardment: (1) *Agrobacterium* injection with the help of a hypodermic needle and (2) opening of an artificial window followed by "spraying" or "coating" the tassel with an *Agrobacterium* solution. For both approaches, it is desirable to remove the *Agrobacteria* by known methods a few days after transformation. For tassels derived from a later developmental stage, microinjection might also represent a suitable transformation technique depending on the plant material and the size of the tissue/organ to be transformed.

Another consideration, especially for *in planta* approaches aiming at the direct harvest of pollen from a transformed plant, is the definition of the best modes to isolate the pollen from the areas transformed, e.g. hit by the bombardment. Four options exist: (1) Harvest the pollen of the whole tassel. (2) Isolate the targeted tassel branches (usually they are shorter and show some signs of damage) by using individual cellophane bags. (3) Cut the non-targeted branches of the tassel and leave only the ones that have been hit. (4) Separate the hit branches for maturation *in vitro.* All four methods yielded pollen, wherein a genetic manipulation/genetic modification could be detected via PCR. Several methods exist for providing molecular evidence that a transgene of interest was indeed integrated into the genome of a pollen, or for proving the successful transient introduction of a genetic construct and its inherited effect in a pollen. PCR turned out to be the most suitable tool for detection, but any suitable method can be used for screening or analyzing a pollen or likewise an ovule of interest. The pollen of the transformed plant could be directly used to pollinate donor plants to assess the next-generation inheritance of the transgene or the targeted genetic manipulation. The produced pollen from maize could be used to pollinate donor plants. The seeds produced were germinated and the progeny were tested for the presence of the transgene or the targeted genetic manipulation by means of PCR or Southern blotting.

### Example 7: Artificial windowing of wheat plants

Several additional experiments have been conducted for testing the applicability of the windowing technology of the present invention with further plants of agronomic interest. One series of experiments was conducted with *Triticum aestivum.* To this end, the development of wheat plants and the meristem and the tassel tissues thereof has been monitored for different wheat varieties. In comparison to maize, the wheat plant has tillers that are at different developmental stages. Consequently, the age of the plant when the immature ear will be ready cannot that easily be determined as for maize. This can, however, be deduced from the developmental state of each tiller. Furthermore, in wheat, the ear has the male and female flowers. Therefore, when targeting a wheat immature ear, there is the chance of targeting the tissues that will produce ovules and pollen. For the purpose of generating transiently or especially stably transformed plants, this is a huge advantage. As detailed in **Figs. 9** **(A-D)** the window opening and thus the creation of an artificially introduced window has been conducted for several wheat plants in a comparable manner as done before with maize plants. Fortunately, the meristem in certain wheat varieties is that big that it can be seen by the eye and no microscopy assistance will be needed in comparison to several other plants including sorghum, where the detection of the meristematic tissue can also be accomplished with the help of microscopy assistance.

The thus exposed wheat meristematic tissues as shown in **Fig. 9** **(A-D)** were subsequently transformed either *in planta* or *in vitro.* For stable transformation, a particle bombardment approach as detailed above for maize, but using a lower shot pressure in the range of 10 to 500 psi, preferably in the range of 50 to 400 psi. As confirmed by microscopy and further by PCR analysis of the differentiated material from the thus transformed meristem, the stable integration of a fluorescent marker protein into the target cells could be detected.

## Claims

1. A method for the *in planta* transformation of a plant or plant material with a genetic construct of interest comprising the following steps:
(i) providing a plant comprising at least one meristematic cell of a meristematic tissue of an immature inflorescence, wherein the meristematic cell is able to differentiate into a gamete of a pollen or of an ovule;
(ii) exposing the at least one meristematic cell of the meristematic tissue of the immature inflorescence;
(iii) providing a genetic construct of interest; and
(iv) transforming the at least one meristematic cell of the meristematic tissue of the immature inflorescence by introducing the genetic construct of interest under suitable conditions to allow the functional integration of the genetic construct of interest into the at least one meristematic cell of the meristematic tissue of the immature inflorescence.

2. The method according to claim 1, wherein the transformation is performed *in vitro* culture free.

3. The method according to claim 1 or 2, wherein the functional integration of the genetic construct of interest into the at least one meristematic cell of the meristematic tissue of the immature inflorescence of step (iv) of claim 1 is performed as
(a) a stable integration so that the integrated genetic construct of interest, or a part thereof, is heritable to a progeny, preferably whereby a transformed gamete of the pollen or of the ovule is generated from the at least one transformed meristematic cell and the transformed gamete of the pollen or of the ovule is fused to a zygote from which the progeny comprising the genetic construct of interest is developed; as transgene, or
(b) a transient introduction allowing a targeted genetic manipulation of the at least one meristematic cell of the meristematic tissue of the immature inflorescence through the genetic construct of interest or products thereof, wherein the targeted genetic manipulation but not the genetic construct of interest, or a part thereof, is heritable to a progeny, preferably whereby a genetic manipulated gamete of the pollen or of the ovule is generated from the transformed at least one meristematic cell and the genetic manipulated gamete of the pollen or of the ovule is fused to a zygote from which the progeny comprising the targeted genetic manipulation is developed.

4. The method according to any one of claims 1 to 3, wherein the method comprises an additional step (v) comprising determining the functional integration of the genetic construct of interest.

5. The method according to any one of the preceding claims, wherein the introduction of the genetic construct of interest according to step (iv) of claim 1 is conducted using a means selected from the group consisting of a device suitable for particle bombardment, including a gene gun, transformation, including transformation using *Agrobacterium spp.* or using a viral vector, microinjection, electroporation, whisker technology, including silicon carbide whisker technology, and transfection, or a combination thereof.

6. The method according to any one of the preceding claims, wherein step (ii) of claim 1 comprises the production of an artificially introduced window in the region, where the meristematic tissue of the immature inflorescence is located, preferably in the shoot axis, in particular in the axil, halm, culm or stem of the plant.

7. The method according to any one of the preceding claims, wherein the plant or the plant material is or is part of a monocotyledonous plant, or is or is part of a dicotyledonous plant.

8. The method according to any one of the preceding claims, wherein the plant or the plant material is or is part of a plant from a species selected from the group consisting of *Hordeum vulgare, Hordeum bulbusom, Sorghum bicolor, Saccharum officinarium, Zea mays, Setaria italica, Oryza minuta, Oriza sativa, Oryza australiensis, Oryza alta, Triticum aestivum, Secale cereale, Malus domestica, Brachypodium distachyon, Hordeum marinum, Aegilops tauschii, Daucus glochidiatus, Beta vulgaris, Daucus pusillus, Daucus muricatus, Daucus carota, Eucalyptus grandis, Nicotiana sylvestris, Nicotiana tomentosiformis, Nicotiana tabacum, Solanum lycopersicum, Solanum tuberosum, Coffea canephora, Vitis vinifera, Erythrante guttata, Genlisea aurea, Cucumis sativus, Morus notabilis, Arabidopsis arenosa, Arabidopsis lyrata, Arabidopsis thaliana, Crucihimalaya himalaica, Crucihimalaya wallichii, Cardamine flexuosa, Lepidium virginicum, Capsella bursa pastoris, Olmarabidopsis pumila, Arabis hirsute, Brassica napus, Brassica oeleracia, Brassica rapa, Raphanus sativus, Brassica juncea, Brassica nigra, Eruca vesicaria subsp. sativa, Citrus sinensis, Jatropha curcas, Populus trichocarpa, Medicago truncatula, Cicer yamashitae, Cicer bijugum, Cicer arietinum, Cicer reticulatum, Cicer judaicum, Cajanus cajanifolius, Cajanus scarabaeoides, Phaseolus vulgaris, Glycine max, Gossypium sp., Astragalus sinicus, Lotus japonicas, Torenia fournieri, Allium cepa, Allium fistulosum, Allium sativum,* and *Allium tuberosum.*

9. The method according to any one of claims 1 to 6, wherein the immature inflorescence is a male inflorescence (tassel) of a *Zea mays* plant.

10. The method according to claim 9, wherein the introduction of the genetic construct of interest as defined in step (iv) of claim 1 is performed at a developmental stage, either during the stamen initiation process or before spikelets are formed on the male inflorescence.

11. A method for manufacturing a transgenic plant comprising the following steps:
(I) providing an *in planta* transformed plant or plant material transformed by a method according to any one of claims 1 to 10;
(II) cultivating the plant or plant material under suitable conditions until the developmental stage of maturity of the inflorescence is achieved,
(III) allowing the transformed gamete of the pollen or of the ovule generated from the transformed at least one meristematic cell to fuse to a zygote comprising the genetic construct of interest as transgene; and
(IV) developing the transgenic plant from the zygote.

12. A method for manufacturing a genetically manipulated plant comprising the following steps:
(I) providing an *in planta* transformed plant or plant material transformed by a method according any one of claims 1 to 10;
(II) cultivating the plant or plant material under suitable conditions until the developmental stage of maturity of the inflorescence is achieved;
(III) allowing the genetically manipulated gamete of the pollen or of the ovule generated from the transformed at least one meristematic cell to fuse to a zygote comprising the targeted genetic manipulation; and
(IV) developing the genetically manipulated plant from the zygote.

13. A plant manufactured by the method of claim 11 or 12 or plant cells, a plant material, or derivatives, or a progeny thereof.

14. A plant, preferably a *Zea mays* plant, comprising an artificially introduced window in a region, where a meristematic tissue of an immature inflorescence is located, preferably in the shoot axis, in particular in the axil, halm, culm or stem of the plant preferably wherein the plant is a plant *in planta* transformed by a method according to any one of claims 1 to 10.

15. Use of a plant according to claim 14, or of a plant *in planta* transformed by a method according to any one of claims 1 to 10, for the manufacture of a transgenic or genetically manipulated plant, plant cell or plant material.
